(19) Europäisches Patentamt
European Patent Office
Office européen des brevets

(11) Veröffentlichungsnummer: 0 222 098
B1

(12) # EUROPÄISCHE PATENTSCHRIFT

(45) Veröffentlichungstag der Patentschrift: **02.05.90**

(21) Anmeldenummer: **86112458.4**

(22) Anmeldetag: **09.09.86**

(51) Int. Cl.⁵: **C 09 B 62/503,**
C 07 C 315/04, C 07 C 317/34,
C 07 C 323/23

(54) **Wasserlösliche Triphendioxazin-Verbindungen, Verfahren zu ihrer Herstellung und ihre Verwendung als Farbstoffe.**

(30) Priorität: **19.09.85 DE 3533411**
**26.07.86 DE 3625347**

(43) Veröffentlichungstag der Anmeldung:
**20.05.87 Patentblatt 87/21**

(45) Bekanntmachung des Hinweises auf die Patenterteilung:
**02.05.90 Patentblatt 90/18**

(84) Benannte Vertragsstaaten:
**BE CH DE FR GB IT LI**

(56) Entgegenhaltungen:
**EP-A-0 053 743**
**EP-A-0 144 580**
**EP-A-0 153 599**

(73) Patentinhaber: **HOECHST AKTIENGESELLSCHAFT**
**Postfach 80 03 20**
**D-6230 Frankfurt am Main 80 (DE)**

(72) Erfinder: **Schwaiger, Günther, Dr.**
**Johannesallee 41**
**D-6230 Frankfurt am Main 80 (DE)**
Erfinder: **Springer, Hartmut, Dr.**
**Am Erdbeerstein 27**
**D-6240 Königstein/Taunus (DE)**
Erfinder: **Helmling, Walter, Dr.**
**Fichtestrasse 29**
**D-6238 Hofheim am Taunus (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 222 098 B1

Courier Press, Leamington Spa, England.

**Beschreibung**

Die vorliegende Erfindung liegt auf dem Gebiet der faserreaktiven Farbstoffe.

Aus der europäischen Patentanmeldungs-Veröffentlichung Nr. 0 141 996 A sind bereits symmetrisch aufgebaute faserreaktive Triphendioxazin-Farbstoffe bekannt, die jedoch noch verbesserungswürdige Eigenschaften besitzen.

Es wurden nunmehr neue wasserlösliche Triphendioxazin-Verbindungen entsprechend der allgemeinen Formel (1)

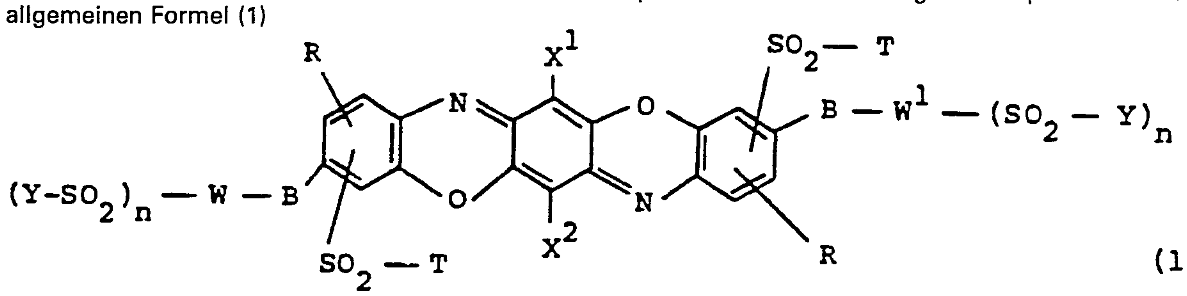

(1)

gefunden, die wertvolle faserreaktive Farbstoffeigenschaften besitzen.

In dieser Formel (1) bedeuten:

T ist eine gegebenenfalls substituierte Alkylgruppe (einschließlich Aralkyl) von 1 bis 6 C-Atomen, bevorzugt von 1 bis 4 C-Atomen, ausgenommen ein Rest der nachstehend definierten Gruppe Y, wobei die Alkylgruppe noch durch Heterogruppen, bevorzugt 1 oder 2 Heterogruppen, die aus Gruppen der Formeln —O—, —S—, —NH— und —N(R')— mit R' der nachstehend angegebenen Bedeutung ausgewählt sind, unterbrochen sein kann, oder ist eine gegebenenfalls substituierte Arylgruppe;

B ist ein Sauerstoff- oder Schwefelatom oder eine Aminogruppe der Formel —NH— oder —N(R')—, in welcher R' eine Alkylgruppe von 1 bis 6 C-Atomen, bevorzugt von 1 bis 4 C-Atomen, wie insbesondere die Methyl- oder Ethylgruppe, ist die substituiert sein kann;

W ist ein bivalenter, gegebenenfalls substituierter aliphatischer, gegebenenfalls durch Alkyl substituierter ($C_5$—$C_{10}$)-cycloaliphatischer, gegebenenfalls durch Alkyl substituierter aliphatisch-($C_5$—$C_8$)-cycloaliphatischer, gegebenenfalls substituierter araliphatischer oder gegebenenfalls substituierter aromatisch-carbocyclischer Rest oder ein mit einem dieser Reste kombinierter Benzotriazolrest, wobei die aliphatischen Reste in W durch Heterogruppen, bevorzugt 1 oder 2 Heterogruppen, unterbrochen sein können, die aus den Gruppen —O—, —S—, —$SO_2$—, —CO—, 1,4-Piperidino, —NH— und —N(R°)— worin R° ein der Bedeutungen von R' hat oder eine Alkanoylgruppe von 2 bis 5 C-Atomen, wie Acetylgruppe, ist, ausgewählt sind, und/oder wobei aliphatische und Arylreste durch eine solche Heterogruppe miteinander verbunden sein können;

$W^1$ hat eine für W angegebene Bedeutung und ist mit W gleich oder von W verschieden;

R ist ein Wasserstoffatom oder ein Alkyl von 1 bis 6 C-Atomen, bevorzugt von 1 bis 4 C-Atomen, wie insbesondere hiervon Methyl und Ethyl, ein Alkoxy von bis 5 C-Atomen, bevorzugt von 1 bis 4 C-Atomen, wie insbesondere hiervon Methoxy und Ethoxy, ein Halogen, wie Fluor und Brom und insbesondere Chlor, Carboxy oder Sulfo;

$X^1$ ist ein Wasserstoffatom oder ein Halogenatom, wie Fluor und insbesondere ein Chlor- oder Bromatom, eine Cycloalkylgruppe von 5 bis 8 C-Atomen, wie die Cyclohexylgruppe, eine Aralkyloxygruppe, eine Alkoxygruppe von 1 bis 4 C-Atomen wie die Methoxygruppe, eine Aryloxygruppe, eine Alkylgruppe von 1 bis 4 C-Atomen, wie die Methylgruppe, eine Arylgruppe, eine Aralkylgruppe, eine Cyangruppe, eine Carboxygruppe, eine Carbalkoxygruppe von 2 bis 5 C-Atomen, wie die Carbomethoxy- oder Carbethoxygruppe, eine Arylaminogruppe, eine Carbamoylgruppe, eine N-Alkyl-carbamoyl-Gruppe oder N,N-Dialkylcarbamoyl-Gruppe mit Alkylresten von jeweils 1 bis 4 C-Atomen, eine N-Aryl-carbamoyl-Gruppe, eine Alkanoylaminogruppe von 2 bis 5 C-Atomen, wie die Acetylaminogruppe, oder eine Aroylaminogruppe, wie die Benzoylaminogruppe, wobei die Arylreste in diesen genannten Substituenten bevorzugt Phenylreste sind, die noch durch 1 oder 2 Substituenten aus der Gruppe Halogen, wie Chlor, Nitro, Alkyl von 1 bis 4 C-Atomen, Alkoxy von 1 bis 4 C-Atomen, Carboxy und Sulfo substituiert sein können und wobei $X^1$ bevorzugt ein Wasserstoffatom, eine Alkanoylaminogruppe von 2 bis 5 C-Atomen, eine Phenoxygruppe, die substituiert sein kann, eine Alkoxygruppe von 1 bis 4 C-Atomen, eine Alkylgruppe von 1 bis 4 C-Atomen und insbesondere bevorzugt ein Chloratom oder Bromatom ist;

$X^2$ ist mit $X^1$ gleich oder von $X^1$ verschieden und hat eine der für $X^1$ angegebenen Bedeutungen;

die Gruppe —$SO_2$—T steht bevorzugt in ortho-Stellung zur Gruppe —B—W—$(SO_2—Y)_n$ bzw. —B—$W^1$—$(SO_2—Y)_n$ gebunden;

n ist die Zahl 1 oder 2, bevorzugt jedoch 1;

Y ist die Vinylgruppe oder eine Ethylgruppe, die in ß-Stellung einen durch ein Alkali eliminierbaren Substituenten enthält;

von den Sulfo- und Sulfatogruppen, die im Molekül (1) enthalten sein können, enthält das Molekül (1) zwingend mindestens eine, bevorzugt mindestens zwei davon.

Die einzelnen, auch zweifach erscheinenden Formelglieder, können zueinander gleiche oder voneinander verschiedene Bedeutungen besitzen.

Ein für $W^1$ erwähnter, mit einem aliphatischen oder aromatischen Rest kombinierter Benzotriazolrest ist beispielsweise ein Rest der allgemeinen Formel (2A)

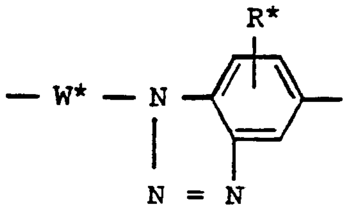

(2A),

in entsprechender Weise ein für W erwähnter, mit einem der aliphatischen oder aromatischen Reste kombinierter Benzotriazolrest beispielsweise ein Rest der allgemeinen Formel (2B)

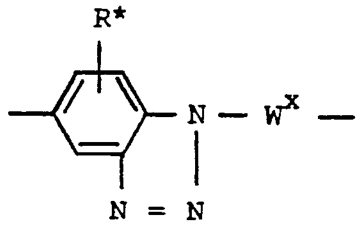

(2B)

in welchen W* und $W^x$ jeweils einen bivalenten, gegebenenfalls substituierten aliphatischen, gegebenenfalls durch Alkyl substituierten ($C_5$—$C_{10}$)-cycloaliphatischen, gegebenenfalls durch Alkyl substituierten aliphatisch-($C_5$—$C_8$)-cycloaliphatischen, gegebenenfalls substituierten araliphatischen oder gegebenenfalls substituierten aromatisch-carbocyclischen Rest bedeuten, wobei die aliphatischen Reste in W* bzw. $W^x$ durch Heterogruppen, bevorzugt 1 oder 2 Heterogruppen, die aus den Gruppen der Formeln —O—, —S—, —$SO_2$—, —CO—, 1,4-Piperidino, —NH— und —N($R^o$)— mit $R^o$ der obengenannten Bedeutung ausgewählt sind, unterbrochen sein können und/oder aliphatische und Arylreste durch eine solche Heterogruppe miteinander verbunden sein können, un in welchen R* jeweils ein Wasserstoffatom, eine Alkylgruppe von 1 bis 4 C-Atomen, wie die Methyl- oder Ethylgruppe, eine Alkoxygruppe von 1 bis 4 C-Atomen, wie die Methoxy- oder Ethoxygruppe, ein Halogenatom, wie Chloratom, oder eine Carboxy- oder Sulfogruppe bedeutet.

Arylreste in den obengenannten oder nachstehend genannten Gruppen sind insbesondere die Phenyl- und Naphthylreste; sie können substituiert sein, wie beispielsweise durch Substituenten aus der Gruppe Alkyl von 1 bis 4 C-Atomen, wie Methyl und Ethyl, Alkoxy von 1 bis 4 C-Atomen, wie Methoxy und Ethoxy, Halogen, wie Chlor und Brom, Sulfo, Carboxy, Sulfamoyl, Carbamoyl, durch Phenyl, Alkyl von 1 bis 4 C-Atomen, Phenylalkyl mit einem Alkylrest von 1 bis 4 C-Atomen und/oder Cycloalkyl von 5 bis 8 C-Atomen mono- oder disubstituiertes Carbamoyl, durch Phenyl, Alkyl von 1 bis 4 C-Atomen, Phenylalkyl mit einem Alkylrest von 1 bis 4 C-Atomen und/oder Cycloalkyl von 5 bis 8 C-Atomen mono- oder disubstituiertes Sulfamoyl, Trifluormethyl, Benzoylamino, Sulfobenzoylamino, Alkanoylamino von 2 bis 5 C-Atomen, Nitro, Amino und gegebenenfalls mono- oder disubstituiertes Amino, wobei dessen Substituenten gegebenenfalls substituierte aliphatische (einschließlich araliphatische), gegebenenfalls substituierte Aryl- und ($C_5$—$C_8$)-Cycloalkyl-Reste sind.

Arylreste in den araliphatischen Resten sind insbesondere Phenylen- und Naphthylenreste; sie können substituiert sein, wie beispielsweise durch Substituenten aus der Gruppe Alkyl von 1 bis 4 C-Atomen, wie Methyl und Ethyl, Alkoxy von 1 bis 4 C-Atomen, wie Methoxy und Ethoxy, Halogen, wie Chlor und Brom, Sulfo, Carboxy, Sulfamoyl, Carbamoyl, durch Phenyl, Alkyl von 1 bis 4 C-Atomen, Phenylalkyl mit einem Alkylrest von 1 bis 4 C-Atomen und/oder Cycloalkyl von 5 bis 8 C-Atomen mono- oder disubstituiertes Carbamoyl, durch Phenyl, Alkyl von 1 bis 4 C-Atomen, Phenylalkyl mit einem Alkylrest von 1 bis 4 C-Atomen und/oder Cycloalkyl von 5 bis 8 C-Atomen mono- oder disubstituiertes Sulfamoyl, Trifluormethyl, Benzoylamino, Sulfobenzoylamino, Alkanoylamino von 2 bis 5 C-Atomen, Nitro, Amino und gegebenenfalls mono- oder disubstituiertes Amino, wobei dessen Substituenten gegebenenfalls substituierte aliphatische (einschließlich araliphatische), gegebenenfalls substituierte Aryl- und ($C_5$—$C_8$)-Cycloalkyl-Reste sind.

Aromatische-carbocyclische Reste sind beispielsweise Phenylen- und Naphthylen- bzw. Phenyl- und Naphthylreste, die substituiert sein können, wie beispielsweise durch Substituenten aus der Gruppe Alkyl von 1 bis 4 C-Atomen, wie Methyl und Ethyl, Alkoxy von 1 bis 4 C-Atomen, wie Methoxy und Ethoxy, Halogen, wie Chlor und Brom, Sulfo, Carboxy, Sulfamoyl, Carbamoyl, durch Phenyl, Alkyl von 1 bis 4 C-Atomen, Phenylalkyl mit einem Alkylrest von 1 bis 4 C-Atomen und/oder Cycloalkyl von 5 bis 8 C-Atomen mono- oder disubstituiertes Carbamoyl, durch Phenyl, Alkyl von 1 bis 4 C-Atomen, Phenylalkyl mit einem Alkylrest von 1 bis 4 C-Atomen und/oder Cycloalkyl von 5 bis 8 C-Atomen mono- oder disubstituiertes Sulfamoyl, Trifluormethyl, Benzoylamino, Sulfobenzoylamino, Alkanoylamino von 2 bis 5 C-Atomen, Nitro, Amino und gegebenenfalls mono- oder disubstituiertes Amino, wobei dessen Substituenten

gegebenenfalls substituierte aliphatische (einschließlich araliphatische), gegebenenfalls substituierte Aryl- und ($C_5$—$C_8$)-Cycloalkyl-Reste sind. Hiervon bevorzugt sind insbesondere solche Phenylen- oder Phenylreste, die durch Substituenten aus der Gruppe Alkyl von 1 bis 4 C-Atomen, Alkoxy von 1 bis 4 C-Atomen und Sulfo und/oder durch eine gegebenenfalls mono- oder disubstituierte Aminogruppe substituiert sein können.

Aliphatische Reste sind beispielsweise Alkylgruppen oder Alkylengruppen von jeweils 1 bis 6 C-Atomen, bevorzugt 1 bis 4 C-Atomen, die substituiert sein können. Substituierte Alkyl- und Alkylengruppen sind beispielsweise solche, die durch 1 oder 2 Substituenten aus der Gruppe Chlor, Alcoxy von 1 bis 4 C-Atomen, Benzoylamino, Sulfobenzoylamino, Alkanoylamino von 2 bis 5 C-Atomen, Hydroxy, Sulfato, Phosphato, Acetyloxy, Sulfo, Carboxy oder gegebenenfalls substituiertes Aryl substituiert sein können. Bevorzugte Substituenten sind hiervon die Carboxy- und Sulfogruppen sowie Sulfatogruppen.

Die Formelglieder W und $W^1$ sowie W* bzw. $W^x$ sind beispielsweise Alkylengruppen von 1 bis 6 C-Atomen, insbesondere von 2 bis 4 C-Atomen, wobei die Alkylenkette durch 1 oder 2 Heterogruppen, die bevorzugt aus den Gruppen —O—, —NH— und —N(R')— mit R' der obigen Bedeutung ausgewählt sind, unterbrochen sein kann, oder ein Alkylen-phenylen-, ein Phenylen-alkylen, ein Phenylen-alkylen-phenylen- oder Alkylen-phenylen-alkylen-Rest, wobei in diesen araliphatischen Resten die Alkylenreste solche von 1 bis 6, bevorzugt 1 bis 4 C-Atomen sind und gegebenenfalls durch die angegebenen Substituenten substituiert und/oder durch eine oder zwei der genannten Heterogruppen unterbrochen sein können und die Benzolkerne jeweils noch durch 1 oder 2 Substituenten substituiert sein können, die aus der Gruppe der Substituenten von Sulfo, Carboxy, Sulfamoyl, Carbamoyl, Methyl, Ethyl, Methoxy, Ethoxy, Nitro, Chlor, Amino und durch gegebenenfalls substituierte aliphatische und/oder gegebenenfalls substituierte Arylreste substituiertes Amino ausgewählt sind, wobei im Falle, daß eine Alkylengruppe durch Heterogruppen unterbrochen ist, deren Alkylenanteile darin bevorzugt solche von 2 oder 3 C-Atomen sind, und wobei die aliphatischen und Arylreste noch durch ein Sauerstoffatom oder eine Gruppe —NH— verbunden sein können. Die Formelglieder W und $W^1$ bzw. W* und $W^x$ sind weiterhin beispielsweise ein Phenylenrest, insbesondere ein meta- oder para-Phenylenrest, der noch durch 1 oder 2 Substituenten aus der Gruppe Sulfo, Carboxy, Sulfamoyl, Carbamoyl, Methyl, Ethyl, Methoxy, Ethoxy, Chlor, Amino und durch gegebenenfalls substituierte aliphatische und/oder gegebenenfalls substituierte Arylreste substituiertes Amino, bevorzugt jedoch durch Sulfo, substituiert sein kann, oder ein gegebenenfalls durch Sulfo substituierter Naphthylenrest.

Bevorzugt ist W bzw. $W^1$, W* und $W^x$ ein Alkylenrest von 2 bis 4 C-Atomen, der durch 1 oder 2 Substituenten, bevorzugt einen Substituenten, aus der Gruppe Sulfo, Sulfato, Carboxy, Phenyl und Sulfophenyl, substituiert sein kann, oder W bzw. $W^1$ ein Phenylenrest, der durch 1 oder 2 Substituenten, bevorzugt einen Substituenten, aus der Gruppe Sulfo, Carboxy, Methyl, Methoxy, Ethoxy und Chlor substituiert sein kann, oder W bzw. $W^1$, W* und $W^x$ sind bevorzugt ein Alkylenphenylen-Rest aus diesen Resten, wobei diese auch durch ein Sauerstoffatom oder eine Gruppe —NH— verbunden sein können.

Die faserreaktiven Gruppen der Formel —$SO_2$—Y sind an aliphatische und/oder aromatische C-Atome, bevorzugt aromatische C-Atome, von W bzw. $W^1$ gebunden.

Reste W* sind beispielsweise der meta- oder para-Phenylenrest, der 1,2-Ethylen-, 1,3-Propylen-, 1,4-Butylen, 1,5-Pentylen-, 1,6-Hexylen-, 2-Methyl-1,3-propylen-, 2-Sulfophenyl-1,3-propylen- und 2-Sulfato-1,3-propylen-Rest, desweiteren beispielsweise ein sulfosubstituierter 1,4-Phenylenrest, ein 1,4- und 1,3-Cyclohexylenrest, ein bivalenter Rest der nachstehend angegebenen Formeln (a*) bis (z*), hiervon bevorzugt der 1,2-Ethylen-, 1,3-Propylen- und 1,4-Butylen-Rest, der Rest der Formeln (a*), ein 2-Sulfophenyl-1,3-propylen- und ein 2-Sulfato-1,3-propylen-Rest und ein sulfosubstituierter 1,4-Phenylenrest:

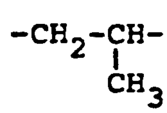
(a*1)

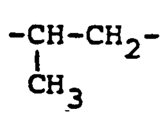
(a*2)

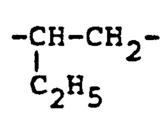
(b*)

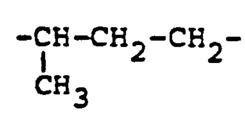
(c*)

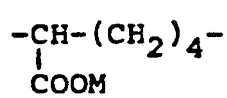
(d*)

$-CH_2-CH_2-O-CH_2-CH_2-$ (e*)

$-CH_2-CH_2-S-CH_2-CH_2-$ (f*)

$-CH_2-CH_2-SO_2-CH_2-CH_2-$ (g*)

$-(CH_2)_3-O-CH_2-CH_2-O-(CH_2)_3-$ (h*)

$-CH_2-CH_2-NH-CH_2-CH_2-$ (j*)

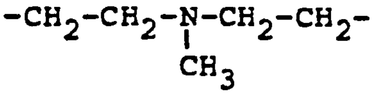 (k*)

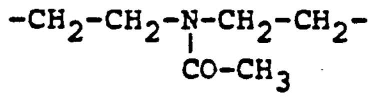 (m*)

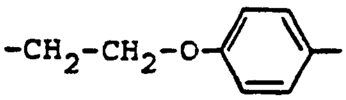 (n*)

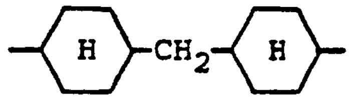 (p*)

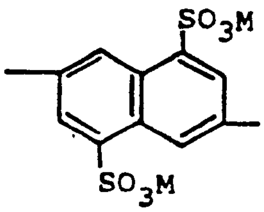 (q*)

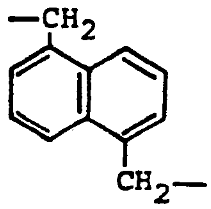 (r*)

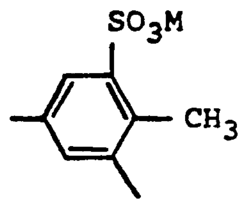 (s*)

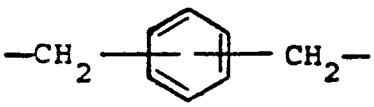 (t*)

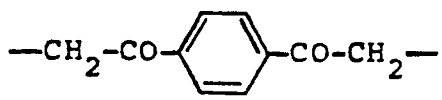 (u*)

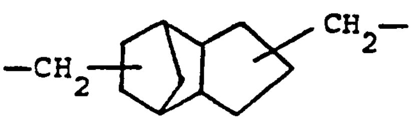 (v*)

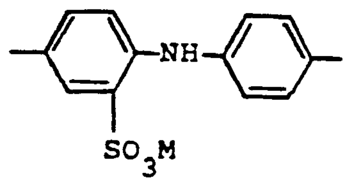 (w*)

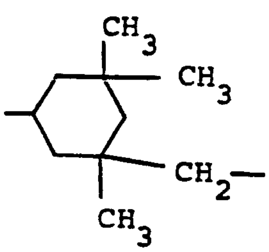 (x*)

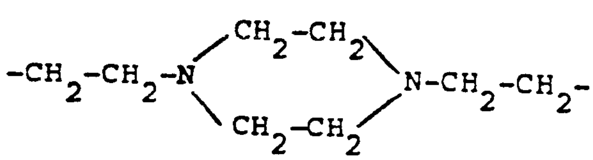 (y*)

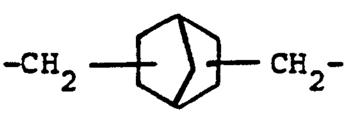 (z*₁)

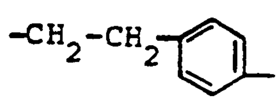 (z*₂)

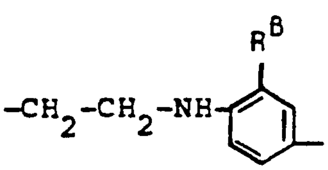 (z*₃)

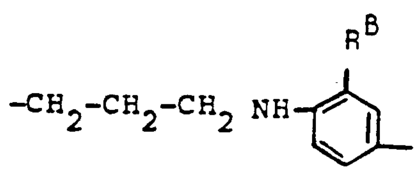 (z*₄)

in welchen M eine der später angegebenen Bedeutungen besitzt und $R^{\beta}$ für Wasserstoff oder Sulfo steht.

Die Reste $W^{x}$ sind beispielsweise die eben für W* genannten, jedoch "spiegelbildlich" angeordneten Gruppen.

Reste $W^{1}$ sind beispielsweise der meta- oder para-Phenylenrest, der 1,2-Ethylen und 1,3-Propylenrest oder ein bivalenter Rest der nachstehend angegebenen Formeln (a) bis (z), wobei $W^{1}$ bevorzugt ein Rest der Formel (b), (g), (m), (q), (x), (y) oder (z) ist:

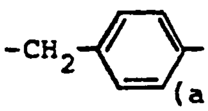
(a)

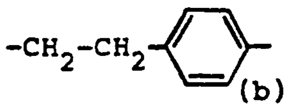
(b)

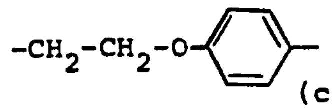
(c)

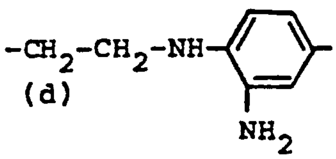
(d)

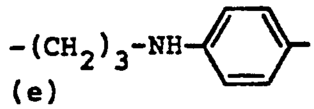
(e)

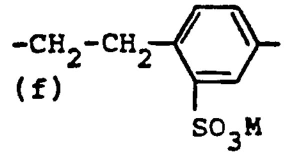
(f)

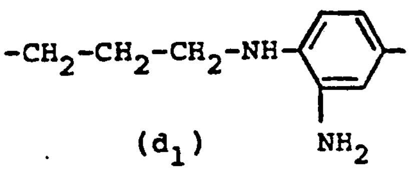
($d_1$)

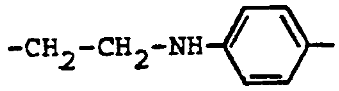
($e_1$)

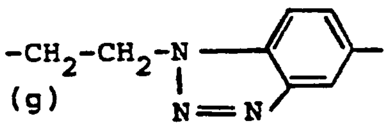
(g)

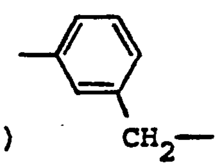
(h)

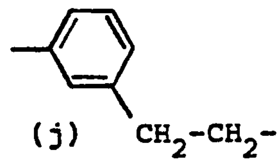
(j)

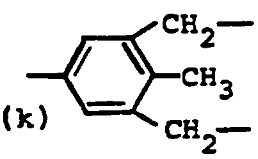
(k)

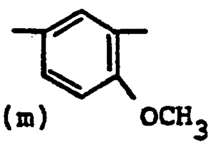
(m)

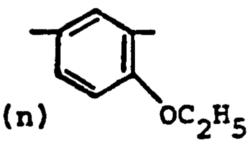
(n)

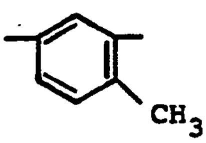
(p)

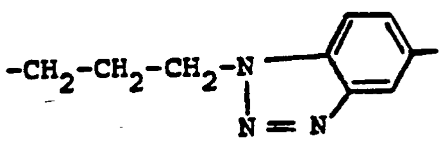
(q)

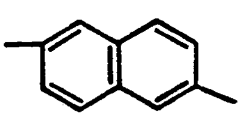
(r)

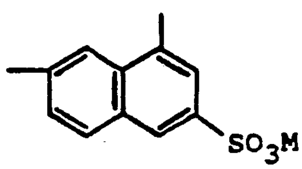
(s)

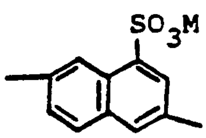
(t)

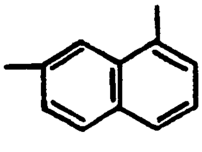
(u)

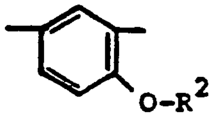
(v)

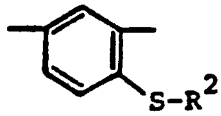
(w)

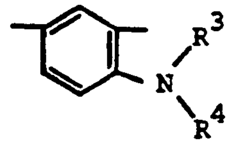
(x)

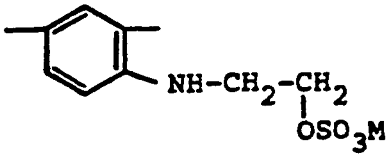
(y)

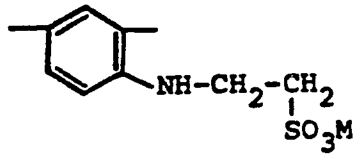
(z)

worin M eine der später angegebenen Bedeutungen bisitzt, $R^2$ eine β-Sulfatoethyl-Gruppe ist, $R^3$ Wasserstoff ist oder Alkyl von 1 bis 4 C-Atomen, das substituiert sein kann, wie beispielsweise durch Sulfo, Carboxy, Sulfato, Phenyl oder Sulfophenyl, bevorzugt Sulfo oder Carboxy, und $R^4$ ein Alkyl von 1 bis 4 C-Atomen bedeutet, das substituiert sein kann, wie durch Sulfo, Carboxy, Sulfato, Phenyl und Sulfophenyl, bevorzugt Sulfo oder Carboxy, oder ein gegebenenfalls substituiertes, wie durch Sulfo oder Carboxy substituiertes, Phenyl ist, wobei beispielsweise die Aminogruppe der allgemeinen Formel —$NR^3R^4$ eine Ethylamino-, Diethylamino-, β-Sulfatoethylamino-, β-Sulfoethylamino, N-Methyl-N-β-sulfoethylamino- oder N-Methyl-N-β-sulfatoethylamino-Gruppe ist.

Die Reste W sind beispielsweise die eben für $W^1$ genannten, jedoch "spiegelbildlich" angeordneten Gruppen.

Der Formelrest T ist bevorzugt ein Alkylrest von 2 bis 6, insbesondere bevorzugt von 2 oder 3 C-Atomen, der durch eine Sulfo- oder Carboxygruppe substituiert ist.

Substituenten, die gemäß dem Formelglied Y in β-Stellung der Ethylgruppe gebunden sind und durch ein Alkali unter Bildung der Vinylgruppe eliminiert werden können, sind beispielsweise Alkanoyloxygruppen von 2 bis 5 C-Atomen, wie die Acetyloxygruppe, Aroyloxygruppen, wie die Benzoyloxy-, Sulfobenzoyloxy- oder Carboxybenzoyloxy-Gruppe, Dialkyl-aminogruppen mit Alkylresten von 1 bis 4 C-Atomen, wie insbesondere die Dimethylamino- und Diethylaminogruppe, Trialkylammoniumgruppen mit Alkyresten von 1 bis 4 C-Atomen wie die Trimethylammoniumgruppe, das Chloratom, das Bromatom, Alkylsulfonyloxygruppen mit Alkyresten von 1 bis 4 C-Atomen, eine Phosphatogruppe, eine Thiosulfatogruppe oder eine Sulfatogruppe. Bevorzugt sind von den Gruppen entsprechend dem Formelglied Y die β-Chlorethyl-β-Phosphatoethyl-, β-Acetyloxyethyl- un β-Thiosulfatoethyl-Gruppe und insbesondere die Vinylgruppe und ganz besonders bevorzugt die β-Sulfatoethyl-Gruppe.

Die beiden Formelreste Y in der allgemeinen Formel (1) können zueinander gleiche oder voneinander verschiedene Bedeutungen haben; bevorzugt besitzen sie die gleiche Bedeutung. Ebenfalls können die Formelreste $(Y—SO_2)_n—W—B—$ und $—B—W^1—(SO_2—Y)_n$ zueinander gleiche oder voneinander verschiedene Bedeutungen besitzen; bevorzugt haben sie die gleiche Bedeutung. Ebenfalls können die Formelreste $—SO_2—T$ zueinander gleiche oder voneinander verschiedene Bedeutungen haben; bevorzugt haben sie die gleiche Bedeutung.

Sulfogruppen sind Gruppen entsprechend der allgemeinen Formel $—SO_3M$, Carboxygruppen sind Gruppen entsprechend der allgemeinen Formel —COOM, Sulfatogruppen sind Gruppen entsprechend der allgemeinen Formel $—OSO_3M$, Thiosulfatogruppen sind Gruppen entsprechend der allgemeinen Formel $—S—SO_3M$ und Phosphatogruppen sind Gruppen entsprechend der allgemeinen Formel $—OPO_3M_2$, wobei M hier, auch später, die folgende Bedeutung besitzt:

M ist ein Wasserstoffatom oder ein Alkalimetall, wie Natrium, Kalium und Lithium, oder das Äquivalent eines Erdalkalimetalls, wie beispielsweise des Calciums, insbesondere jedoch ein Alkalimetall.

In der allgemeinen Formel (1) ist R bevorzugt ein Wasserstoffatom, desweiteren B bevorzugt eine Aminogruppe —NH—, desweiteren der Rest $W^1$ bevorzugt ein Rest der obigen Formel (b), (m), (y) oder (z) und W bevorzugt ein entsprechender Rest mit "spiegelbildlicher" Struktur; bevorzugt ist weiterhin der Formelrest T eine β-Sulfoethyl-Gruppe.

Besonders hervorzuheben sind von den erfindungsgemäßen Triphendioxazin-Verbindungen diejenigen, die der allgemeinen Formel (1a)

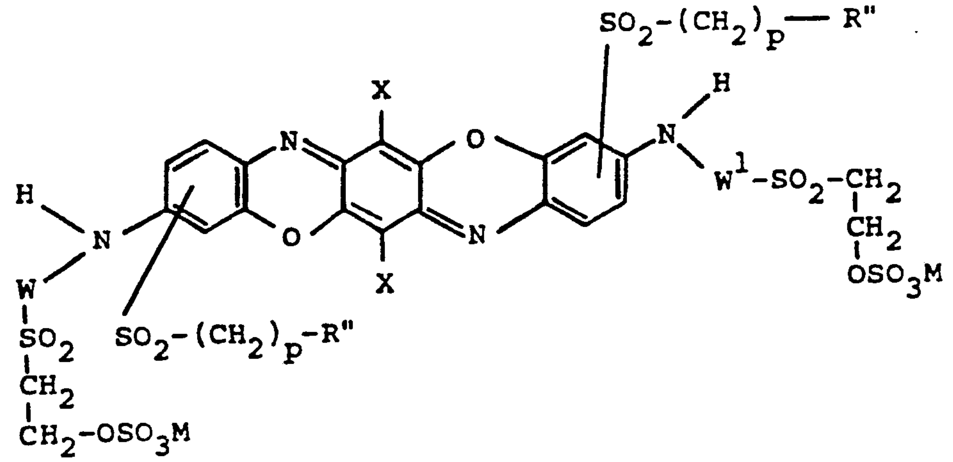

(1a)

entsprechen, in welcher

X für ein Bromatom oder bevorzugt ein Chloratom steht,

M ein Wasserstoffatom oder bevorzugt ein Alkalimetallatom, wie insbesondere Natrium, ist,

P für die Zahl 2 oder 3, bevorzugt 2, steht,

R'' eine Sulfo, Sulfato oder Carboxy, bevorzugt Sulfogruppe bedeutet, im Falle von p = 2 jedoch keine Sulfatogruppe bedeutet,

$W^1$ einen Phenylenrest, bevorzugt meta-Phenylenrest, darstellt, der durch Methyl, Ethyl, Chlor, Methoxy oder Ethoxy substituiert sein kann, bevorzugt durch eine Methoxygruppe substituiert ist, wie ein Rest der Formel (m), oder eine Gruppe der Formel (2a), (2b) oder (2c)

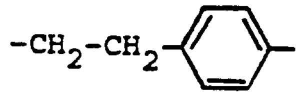 (2a) 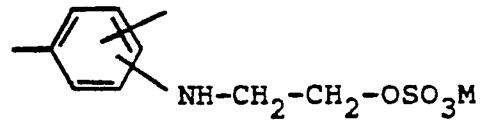 (2b)

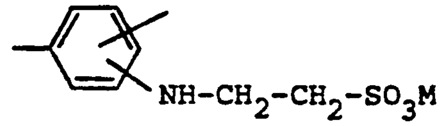 (2c)

oder der obigen Formel (g) oder (q) mit M der obengenannten Bedeutung ist, wobie in den Formeln (2b) und (2c) der Phenylenrest bevorzugt ein meta-Phenylenrest ist und die Aminogruppe in para-Stellung zur anderen, dem Formelrest B entsprechenden Aminogruppe gebunden ist, und

W einen solchen für $W^1$ erwähnten, jedoch "spiegelbildlich" angeordneten Rest bedeutet.

Die neuen Dioxazinverbindungen können sowohl in saurer Form als auch in Form ihrer Salze vorliegen. Bevorzugt sind sie in Form der Salze, insbesondere der Alkalimetallsalze, und finden auch bevorzugt in Form dieser Salze Verwendung zum Färben (hier und im folgenden im allgemeinen Sinne und einschließlich des Bedruckens) verstanden von hydroxy- und/oder carbonamidgruppenhaltigen Materialien, insbesondere Fasermaterialien.

Die vorliegende Erfindung betrifft weiterhin Verfahren zur Herstellung der obengenannten und definierten Verbindungen der allgemeinen Formel (1). Diese sind dadurch gekennzeichnet, daß man eine Verbindung der allgemeinen Formel (3)

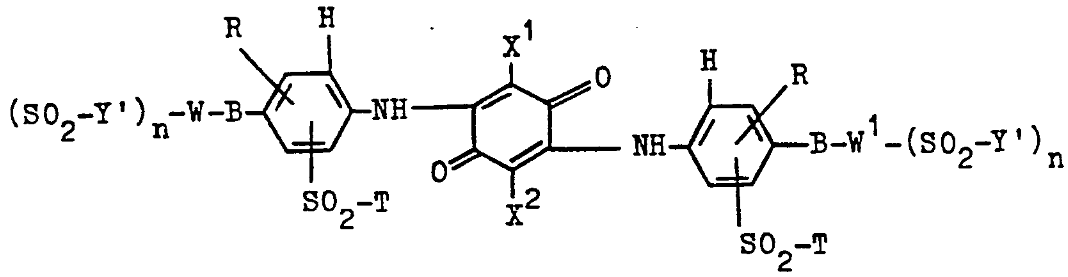 (3)

(in welcher Y' die Vinylgruppe, die β-Hydroxy-ethyl-Gruppe oder eine Ethylgruppe, die in β-Stellung einen durch ein Alkali eliminierbaren Substituenten, wie einen der für Y genannten enthält, bevorzugt die β-Hydroxyethyl-Gruppe ist, und n, R, B, W, $W^1$, T, $X^1$ und $X^2$ die obengenannten Bedeutungen haben, wobei substituiert Alkygruppen in diesen Resten auch hydroxysubstituierte Alkylgruppen sein können und die Gruppen $-SO_2-T$ bevorzugt in ortho-Stellung zur Gruppe $(Y'-SO_2)_n-W-B-$ bzw. $-B-W^1-(SO_2-Y')_n$ gebunden sind) in saurem Medium und vorzugsweise in Gegenwart eines Oxidationsmittels zum Triphendioxazin cyclisiert. Die Umsetgzung erfolgt in an und für sich bekannter Verfahrensweise, so beispielsweise in Schwefelsäure oder Schwefeltrioxid enthaltender Schwefelsäure als Reaktionsmedium, wobei als Oxidationsmittel Schwefeltrioxid, Ammonium- oder Alkalipersulfate, Jod oder anorganische Jodverbindungen in Gegenwart von Oleum, Natriumborat, vorzugsweise jedoch Natrium- oder Kaliumperoxodisulfat (entsprechend den Formeln $Na_2S_2O_8$ bzw. $K_2S_2O_8$), verwendet werden. Solche Verfahrensweisen sind bspw. aus der britischen Patentschrift Nr. 1 589 915 und der europäischen Patentanmeldungs-Veröffentlichung Nr. 0 141 359A bekannt.

Vorzugsweise führt man die Umsetzung in konzentrierter Schwefelsäure, wie 96- bis bevorzugt 100 %iger Schwefelsäure und insbesondere in Schwefeltrioxid enthaltender Schwefelsäure (Oleum), wie bis zu 50 gew.-%igem Oleum, durch. Die Reaktionstemperatur wird zwischen 0 und 80°C gewählt. Das als Reaktionsmedium und Agenz verwendete Oleum besitzt in der Regel einen Gehalt von 5 bis 30 Gew.-%, bevorzugt 10 bis 20 Gew.-% an Schwefeltrioxid. Bei Zusatzvon Peroxodisulfat als Oxidationsmittel führt man die Cyclisierung zwischen 0 und 40°C, bevorzugt zwischen 15 und 25°C durch. Bei Verwendung von Oleum/Peroxodisulfat soll die Reaktionstemperatur 30°C nicht überschreiten. Bevorzugt ist 10 bis 20%iges Oleum unter Verwendung einer zur Verbindung (3) äquivalenten Menge Peroxodisulfat. Bei Jod als Oxidationsmittel wird dieses in katalytischen Mengen in 10 bis 50%igem Oleum eingesetzt; hier liegt die Reaktionstemperatur in der Regel zwischen 0 und 40°C.

Gegebenenfalls vor oder gleichzeitig mit der Cyclisierung oder erst nach der Cyclisierungsreaktion können gegebenenfalls vorhandene Hydroxyalkylgruppen, wie beispielsweise die β-Hydroxyethyl-Gruppe des Formelrestes Y', mittels eines Sulfatisierungs- oder Phosphatierungsmittels, wie 96—100%iger Schwefelsäure oder Schwefeltrioxid enthaltender Schwefelsäure bzw. Polyphosphorsäure, in die entsprechenden β-Sulfatoalkyl- bzw. β-Phosphatoalkyl-Gruppen verestert werden. Wird also der Ringschluß in Schwefelsäure oder Oleum als Reaktionsmedium ausgeführt, werden Hydroxygruppen, die an einem Alkyrest des Moleküls gebunden sind, wie beispielsweise die oben bereits erwähnten β-Hydroxyethyl-Gruppen des Formelrestes Y' oder Hydroxyalkyl-Gruppen der Formelreste W bzw. $W^1$ und T, in die entsprechenden Sulfatoalkylgruppen übergeführt.

Bei Temperaturen oberhalb von etwa 35°C, wie bei Temperaturen zwischen 40 und 60°C, lassen sich erfindungsgemäß auch Sulfogruppen in die aromatischen Ringe des Triphendioxazins (die entsprechenden Arylreste von W, $W^1$, T, $X^1$ und $X^2$ eingeschlossen) einführen.

Verbindungen der Formel (1) mit Y gleich einer β-Sulfatoethyl-Gruppe können anschließend gemäß bekannten Verfahrensweisen in andere erfindungsgemäße Verbindungen der Formel (1) übergeführt werden, in welcher Y für die Vinylgruppe oder eine Ethylgruppe mit einem anderen in β-Stellung befindlichen alkalisch eliminierbaren Substituenten stehen, übergeführt werden.

Die Verbindungen der allgemeinen Formel (3) können analog bekannten Verfahrensweisen durch Umsetzung einer Verbindung der allgemeinen Formel (4)

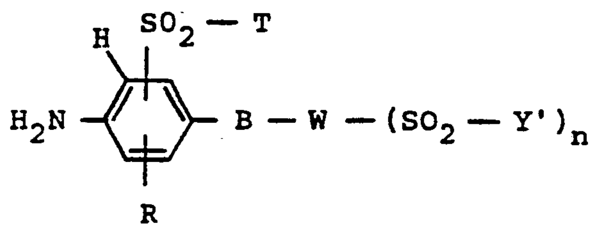

(4)

(bzw. solchen mit $W^1$ statt W), in welchen Y' die obengenannte Bedeutung besitzt und bevorzugt die β-Hydroxyethyl-Gruppe ist und n, R, B, W, $W^1$ und T die obengenannten Bedeutungen haben, wobei substituierte Alkylgruppen in diesen Resten auch hydroxysubstituierte Alkylgruppen sein können und die Gruppen $-SO_2-T$ bevorzugt in ortho-Stellung zur Gruppe $(Y'-SO_2)_n-W-B-$ bzw. $-B-W^1-(SO_2-Y')_n$ gebunden sind, mit einer 1,4-Benzochinon-Verbindungen der allgemeinen Formel (5)

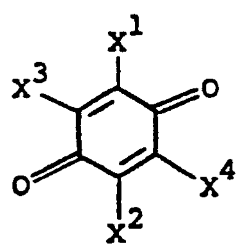

(5)

in welcher $X^1$ und $X^2$ die obengenannten Bedeutungen haben und $X^3$ und $X^4$ zueinander gleich oder voneinander verschieden sind und jedes für ein Wasserstoffatom, eine Alkoxygruppe von 1 bis 4 C-Atomen, wie insbesondere die Methoxygruppe, oder für eine Phenoxygruppe steht oder bevorzugt ein Halogenatom, wie Fluoratom, besonders Bromatom und insbesondere Chloratom, ist wobei $X^3$ und $X^4$ auch eine zu $X^1$ und $X^2$ gleiche Bedeutung haben können, hergestellt werden.

Die Umsetzung einer Verbindung der allgemeinen Formel (4) oder zweier verschiedener Aminoverbindugen der allgemeinen Formel (4), jeweils in zusammen 2-fach äquivalenter Menge, mit einem Äquivalent einer Verbindung der allgemeinen Formel (5) zur Verbindung der allgemeinen Formel (3) erfolgt analog bekannten Verfahrensweisen, die beispielsweise in K. Venkataraman, "The Chemistry of Synthetic Dyes", Band V, S. 419—427 (1971), und in Ullmanns Encyklopädie der technischen Chemie, 4. Aufl., Band 8, S. 240 + 241 (1974) sowie in der britischen Patentanmeldungs-Veröffentlichen Nr. 2 019 872, in der deutschen Offenlegungsschrift Nr. 28 23 828 und in der europäischen Patentanmeldungs-Veröffentlichung Nr. 0 141 996A beschrieben sind. Beispielsweise kann die Umsetzung in wäßrigem Medium oder in wäßrig-organischem Medium oder in rein organischem Medium erfolgen, wobei die organischen Lösemittel polare aprotische und protische Lösungsmittel darstellen, wie beispielsweise niedere Alkanole, wie Methanol und Ethanol, und halogenierte Benzole, wie o-Dichlorbenzol. Bevorzugt wird das Chinon der Formel (5) jedoch in einem mehr oder weniger stärkeren Überschuß eingesetzt, der in der Regel 2—20% beträgt, aber auch, abhängig vom gewählten Chinon, bis zu 100% oder mehr betragen kann. Die Umsetzung der Amine (4) mit den Chinonen (5) kann bei einer Temperatur zwischen 20 und 100°C, vorzugsweise zwischen 50 und 70°C, in Gegenwart eines säurebindenden Mittels, wie beispielsweise eines Alkali- oder Erdalkalicarbonate oder -acetats, so beispielsweise Natriumacetat, Natriumcarbonat oder Natriumbicarbonat, oder eines Alkali- oder Erdalkalihydroxids, wie Natriumhydroxid, oder eines Oxids eines Erdalkalimetalls, wie beispielsweise Magnesiumoxid, durchgeführt werden. Sofern in einem wäßrigen oder

wäßrig-organischen Medium gearbeitet wird, wird ein pH-Bereich zwischen 4 und 7, vorzugsweise zwischen 5,5 und 6,5 eingestellt.

Die Anilin-Ausgangsverbindungen der allgemeinen Formel (4) sind bisher noch nicht bekannt. Die Erfindung betrifft somit auch diese Verbindungen, Verfahren zu deren Herstellung und ihre Verwendung zur Synthese von Farbstoffen. Sie lassen sich analog bekannten Verfahrensweisen der Umsetzung von Nitrochlorbenzolen mit Aminen herstellen, so beispielsweise durch Umsetzung eines Chlornitrobenzols der allgemeinen Formel (6)

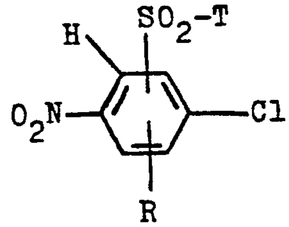

(6)

in welcher R und T die obengenannten Bedeutungen besitzen, mit einem Amin der allgemeinen Formel (7)

$$H—B—W—(SO_2—Y')_n \quad (7)$$

in welcher B, W, Y' und n die obengenannten Bedeutungen haben, in Wasser oder einem organischen Medium, wie einem Alkanol, beispielsweise Methanol, Dioxan und Toluol, oder in einem Gemisch von Wasser und von wassermischbaren organischen Lösemitteln, unter Zusatz eines basischen, säurebindenden Mittels, wie beispielsweise Kaliumcarbonat, Magnesiumoxid, Natriumcarbonat oder Natriumhydroxid oder auch Triethylamin oder Triethanolamin, bei einer Temperatur zwischen 20 und 120°C, insbesonder zwischen 50 und 120°C, gegebenenfalls unter Druck, vorzugsweise zwischen 70 und 120°C. In wäßrigem Medium wird ein pH-Wert zwischen 6 und 12, bevorzugt zwischen 8 und 10, eingehalten.

Die auf diese Weise erhältlichen und ebenfalls neuen und erfindungsgemäßen Nitro-anilin-Verbindungen der allgemeinen Formel (8)

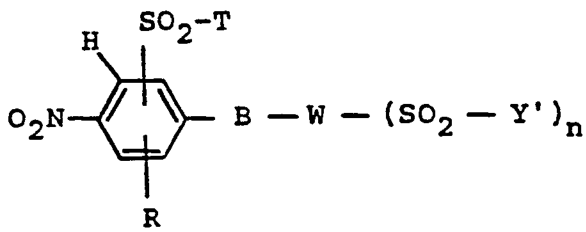

(8)

in welcher R, T, B, W, Y' und n die obengenannten Bedeutungen haben, können sodann nach an und für sich üblichen Methoden der Reduktion von aromatischen Nitrogruppen mittels Wasserstoff und einem metallischen Katalysator, wie einem Palladium-, Platin- oder Raney-Nickelkatalysator, unter Druck im Autoklaven oder mittels der Reduktion nach Béchamp bei Verwendung von Eisenspännen zur Verbindung entsprechend der allgemeinen Formel (4), in welcher Y' bevorzugt für die β-Hydroxy-ethyl-Gruppe steht, synthetisiert werden. Aus den β-Hydroxy-ethylsulfonyl-Verbindungen der allgemeinen Formel (4) lassen sich die entsprechenden faserreaktiven Ausgangsverbindungen (4), in denen Y' für die Vinylgruppe oder eine Ethylgruppe, die in β-Stellung einen durch Alkali eliminierbaren Substituenten enthält, steht, in allgemein bekannter Verfahrensweise der Überführung der β-Hydroxyethylsulfonyl-Gruppe in solche Gruppen herstellen. Bevorzugt wird die β-Hydroxy-ethylsulfonyl-Gruppe in die β-Sulfatoethylsulfonyl-Gruppe übergeführt.

Chlornitrobenzolverbindungen der allgemeinen Formel (6) lassen sich beispielsweise analog den Synthesemöglichkeiten zur Herstellung von Sulfonen, wie beispielsweise in Houben-Weyl, Methoden der Organischen Chemie, Band IX, S, 231ff. (1955) und Band E11, S. 1132ff. (1985), herstellen, so über das Sulfinsäure-Derivat, wie in an und für sich bekannter Verfahrensweise durch Reduktion einer entsprechenden 1-Chlor-4-nitro-benzolsulfochlorid-Verbindung mittels Natriumsulfit zur entsprechenden Sulfinsäure und anschließende Oxalkylierung der Sulfinsäure zur β-Hydroxyalkylsulfonyl-Verbindung, die anschließend sulfatisiert wird. Die Sulfatogruppe kann sodann nach üblichen Methoden mittels Natriumsulfit in die Sulfogruppe übergeführt werden. So kann beispielsweise das 2-(β-Sulfoethylsulfonyl)-4-nitro-chlorbenzol durch Umsetzung von 2-(β-Sulfatoethylsulfonyl)-4-nitro-chlorbenzol, das nach der oben angegebenen Methode aus 1-Chlor-4-nitro-benzolsulfochlorid erhältlich ist, mit einer wäßrigen Natriumsulfitlösung bei einer Temperatur zwischen 30 und 50°C und einem pH-Wert zwischen 6 und 9,5 hergestellt werden.

Aus den Sulfinsäure-Verbindungen lassen sich auch die carboxysubstituierten Alkysulfon-Verbindungen beispielsweise durch Umsetzung mit Chloressigsäure, Acrylsäure oder Crotonsäure herstellen.

Einige der Verbindungen der allgemeinen Formel (6) können auch durch Nitrierung einer entsprechenden Alkylsulfonyl- oder Arylsulfonyl-chlorbenzol-Verbindung in an und für sich üblicher Weise gewonnen werden, so beispielsweise analog den Angaben des Beispieles 5 der deutschen Patentschrift 859 462.

Anilin - Ausgangsverbindungen der allgemeinen Formel (4) sind beispielsweise 3 - (β - Carboxyethylsulfonyl) - 4 - β - [4' - (β' - hydroxyethylsulfonyl) - phenyl] - ethylamino - anilin, 3 - (β - Sulfoethylsulfonyl) - 4 - β -[5' - (β - hydroxyethylsulfonyl) - benzotriazol - 1' - yl] - ethylamino - anilin, 3 - (β - Sulfoethylsulfonyl) - 4 - γ - [5' - β - hydroxyethylsulfonyl) - benzotriazol-1'-yl] - propylamino - anilin, 3- (β - Sulfo - propylsulfonyl) - 4 - β - [4' - (β' - hydroxyethylsulfonyl) - phenyl] - ethylamino - anilin, 3 - (β - Sulfoethylsulfonyl) - 4 - [3' - β - hydroxy - ethylsulfonyl - 4 - (N - methyl - N - β-sulfoethyl) - amino - phenylamino] - anilin, 3 - (β - Sulfoethylsulfonyl) - 4 - (3' - β - hydroxyethylsulfonyl - 4' - ethylamino] - phenylamino - anilin, 3 - (β - Sulfoethyl - sulfonyl) - 4 - [β - (β' - hydroxyethylsulfonyl) - ethylamino] - anilin, 3 - (β - Sulfoethylsulfonyl) - 4 - [γ - (β' - hydroxyethyl - sulfonyl) - propylamino] - anilin, 3 - (β - Sulfoethylsulfonyl) - 4 - [(4' - β - hydroxyethylsulfonyl - phenyl) - methylamino] - anilin, 3 - (β - Sulfoethylsulfonyl) - 4 - [β - (4' - β' - hydroxyethylsulfonyl - phenoxy) - ethylamino] - anilin, 3 - (β - Sulfoethylsulfonyl) - 4 - [γ - (2' - amino - 4' - β' - hydroxyethylsulfonyl - phenylamino] - propylamino - anilin, 3 - (β-Sulfoethylsulfonyl) - 4 - [3' - (β - hydroxyethylsulfonyl) - phenylamino] - anilin, 3 - [β - Sulfoethylsulfonyl) - 4 - [3' - (β - hydroxyethylsulfonyl) - 4 - methyl - phenyl - amino] - anilin, 3 - (β - Sulfoethylsulfonyl) - 4 - [6' - (β - hydroxyethylsulfonyl) - 8' - sulfo - naphth - 2' - yl - amino] - anilin, 3 - (β - Sulfoethylsulfonyl) - 4 - [8' - (β -hydroxyethylsulfonyl) - 6' - sulfo - naphth - 2' - yl - amino] - anilin, 3 - [β - Sulfoethylsulfonyl) - 4 - [6', 8' - Di - (β - hydroxyethylsulfonyl) - naphth - 2' - yl - amino] - anilin und insbesondere 3 - (β - Sulfoethylsulfonyl) - 4 - [β-(4' - β' - hydroxyethylsulfonyl - phenyl) - ethylamino] - anilin, 3 - (β - Sulfoethylsulfonyl) - 4 - [3' - [β - hydroxyethylsulfonyl) - 4 - methoxy - phenylamino] - anilin, 3 - (β - Sulfoethylsulfonyl) - 4 - (3' - (β - hydroxyethylsulfonyl) - 4' - (β - hydroxyethylamino) - phenylamino] - anilin und 3 - (β - Sulfoethylsulfonyl) - 4 - [3' - (β - hydroxyethylsulfonyl) - 4' - [β - sulfoethylamino) - phenylamino] - anilin.

Die als Ausgangsverbindung dienenden Benzochinone der allgemeinen Formel (5) sind in der Literatur zahlreich bekannt. Verbindungen dieser Art sind beispielsweise 1,4 - Benzochinon, 2 - Methyl - 1,4 - benzochinon, 2 - Ethyl - 1,4 - benzochinon, 2 - n - Propyl - 1,4 - benzochinon, 2 - Isopropyl - 1,4 - benzochinon, 2 - (β - Ethoxyethyl) - 1,4 - benzochinon, 2 - Phenyl - 1,4 - benzochinon, 2 - (4' - Methylphenyl) - 1,4 - benzochinon, 2 - (4' - Methoxyphenyl) - 1,4 - benzochinon, 2 - (3' - Chlorophenyl) - 1,4 - benzochinon, 2 - (4' - Nitrophenyl) - 1,4 - benzochinon, 2,5 - Dimethyl - 1,4 - benzochinon, 2 - Methyl - 5 - ethyl - 1,4 - benzochinon, 2 - Methyl - 3 - chloro - 1,4 - benzochinon, 2 - Methyl - 6 - chloro - 1,4 - benzochinon, 2 - Methyl - 3,5 - dichloro - 1,4 - benzochinon, 2 - Methyl - 3,5,6 - tribromo - 1,4 - benzochinon, 2 - (4' - Methylphenoxy) - 3,6 - dibromo - 1,4 - benzochinon, 2 - (3' - Methylphenoxy) - 3,6 - dibromo - 1,4 - benzochinon, 2 - Methyl - 3,5,6 - trichloro - 1,4 - benzochinon, 2 - Methyl - 3 - chloro - 5 - bromo - 1,4 - benzochinon, 2 - Methyl - 3,6 - dichloro - 1,4 - benzochinon, 2 - Methyl - 3,6 - dichloro - 5 - bromo - 1,4 - benzochinon, 2 - Phenyl - 3,6 - dichloro - 1,4 - benzochinon, 2 - (4' - Methoxyphenyl) - 3,6 - dichloro - 1,4 - benzochinon, 2 - (4' - Chlorophenyl) - 3,6 - dichlor - 1,4 - benzochinon, 2 - (4' - Nitrophenyl) - 3,6 - dichloro - 1,4 - benzochinon, 2 - (4' - Nitrophenyl) - 3,5,6 - trichloro - 1,4 - benzochinon, 2,5 - Dimethyl - 3,6 - dibromo - 1,4 - benzochinon, 2,5 - Dimethyl - 3 - chloro - 1,4 - benzochinon, 2 - Methyl - 5 - n - propyl - 6 - bromo - 1,4 - benzochinon, 2 - Methyl - 5 - isopropyl - 3 - chloro - 1,4 - benzochinon, 2 - Methyl - 5 - isopropyl - 6 - bromo - 1,4 - benzochinon, 2 - (2' - Chlorophenyl) - 3,5,7 - tribromo - 1,4 - benzochinon, 2 - Methyl - 3 - methoxy - 1,4 - benzochinon, 2,3,5,6 - Tetramethoxy - 1,4 - benzochinon, 2,3,5,6 - Tetraphenoxy - 1,4 - benzochinon, 2,3,5,6 - Tetra - (4' - methylphenoxy) - 1,4 - benzochinon, 2,3,5,6 - Tetra - (4' - methoxyphenoxy) - 1,4 - benzochinon, 2,3,5,6 - Tetra - (4' - chlorophenoxy) - 1,4 - benzochinon, 2,3,5,6 - Tetra - 4 - (3' - methyl - 4' - chlorophenoxy) - 1,4 - benzochinon, 2 - Ethyl - 3,6 - dimethoxy - 1,4 - benzochinon, 2 - Chloro - 3,6 - dimethoxy - 1,4 - benzochinon, 2,3,5 - Trimethoxy - 1,4 - benzochinon, 2,5 - Dimethyl - 3,6 - dimethoxy - 1,4 - benzochinon, 2,5 - Dimethyl - 3,6 - dimethoxy - 1,4 - benzochinon, 2 - Methyl - 3,6 - dimethoxy - 1,4 - benzochinon, 2 - Methyl - 5,6 - dimethoxy - 1,4 - benzochinon, 2 - Ethyl - 3,6 - dimethoxy - 1,4 - benzochinon, 2 - Chloro - 3 - n - propyl - 5 - methoxy - 1,4 - benzochinon und 2 - Chloro - 3,5 - dimethoxy - 1,4 - benzochinon, 2,3,5,6 - Tetrafluor - 1,4 - benzochinon sowie bevorzugt 2,3,5,6 - Tetrabrom - 1,4 - benzochinon und insbesondere 2,3,5,6 - Tetrachlor - 1,4 - benzochinon.

Ausgangsverbindungen der oben angegebenen und definierten allgemeinen Formel (7), die den faserreaktiven Rest der Vinylsulfon-Reihe enthalten bzw. deren β-Hydroxyethylsulfonyl - Derivat darstellen, sind ebenfalls in der Literatur bekannt oder lassen sich analog den in der Literatur beschreibenen Verfahrensweisen herstellen. Solche Verbindungen sind bevorzugt β - (4 - β' - Hydroxyethylsulfonyl - phenyl) - ethylamin, 3 - (β - Hydroxyethylsulfonyl) - 4 - methoxy - anilin, 3 - (β - Hydroxyethylsulfonyl) - 4 - (β - hydroxyethylamino) - anilin und 3 - (β - Hydroxyethylsulfonyl) - 4 - (β - sulfoethylamino) - anilin, desweiteren β - [5 - (β - Hydroxyethylsulfonyl) - benzotriazol - 1 - yl] - ethylamin, γ - [5 - β - Hydroxyethylsulfonyl) - benzotriazol - 1 - yl] - n - propyl - amin, die ω - [5 - (β - Hydroxyethylsulfonyl) - benzotriazol - 1 - yl] - $C_4$ - $C_6$ - n - alkylamine, β - [5 - (β - Hydroxyethylsulfonyl) - benzotriazol - 1 - yl] - ethanol, die ω - [5 - (β - Hydroxyethylsulfonyl) - benzotriazol - 1 - yl] - $C_3$ - $C_6$ - n - alkanole, β - [5 - (β - Hydroxyethylsulfonyl) - benzotriazol - 1 - yl] -

n - propylamin, 4 - [5' - (β - Hydroxyethylsulfonyl) - benzotriazol - 1' - yl] - cyclohexylamin, β - {4 - [5' - (β - Hydroxyethylsulfonyl) - benzotriazol - 1' - yl] - phenylamino} - ethylamin, β - {4 - [5' - (β - Hydroxyethylsulfonyl) - benzotriazol - 1' - yl] - phenylamino} - ethanol, 4 - [5' - (β - Hydroxyethylsulfonyl) - benzotriazol - 1' - yl] - phenethylamin, β - (4 - β - Hydroxyethylsulfonylphenyl) - ethylamin, β - (4 - β' - Hydroxyethylsulfonyl) - benzylamin, β - (2 - Sulfo - 4 - β' - hydroxyethylsulfonyl - phenyl) - ethylamin und die anderen aus den Beispielen zahlreich ersichtlichen Aminoverbindungen bzw. deren β - Hydroxyethylsulfonyl - Abkömmlinge.

Ausgangsverbindungen der allgemeinen Formel (7), die den Benzotriazolrest enthalten, also Verbindungen der allgemeinen Formel (7a)

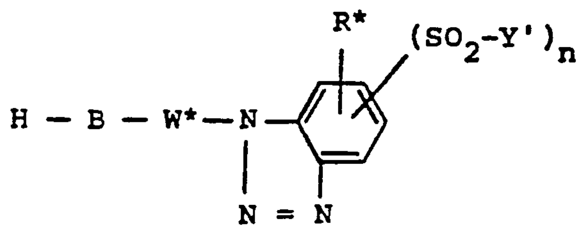

(7a)

in welcher B, W*, Y' und n die obengenannten Bedeutungen haben, sind noch nicht beschrieben. Sie können analog bekannten Verfahrensweisen hergestellt werden, indem man eine Verbindungen der allgemeinen Formel (9)

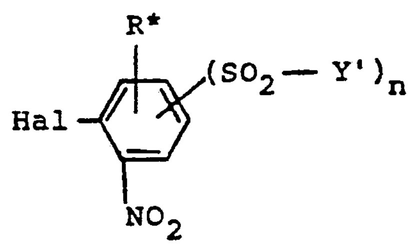

(9)

in welcher R*, Y' und n die obengenannten Bedeutungen haben und Hal für ein Fluor- oder Bromatom steht, mit einer Verbindung der allgemeinen Formel A—B—W*—$NH_2$, in welcher A ein Wasserstoffatom oder Acylrest, wie ein Acetyl- oder Benzoylrest, ist und B und W* die obengenannten Bedeutungen haben und B bevorzugt de Gruppe NH ist, in einem für diese Reaktanten geeigneten Lösemittel in Gegenwart eines säurebindenden Mittels bei einer Temperatur zwischen 30 und 120°C, vorzugsweise zwischen 70 und 90°C, umsetzt.

Ausgangsverbindungen entsprechend der Formel A—B—W*—$NH_2$ sind beispielsweise 1,2-Diamino - ethan, 1,3 - Diamino - propan, 1,4 - Diamino - butan, 1,5 - Diamino - pentan, 1,6 - Diamino - hexan, 1,2 - Diamino - propan, 1,2 - Diamino - butan, 1,3 - Diamino - butan, die N-Acyl-, wie die N - Acetyl- und N - Benzoyl - Verbindungen, des 1 - Amino - 3 - methylamino - propans, 1,3 - Diamino - 2 - methyl - propan, 1,3 - Diamino - 2 - hydroxy - propan, 1,5 - Diamino - 2 - carboxy - pentan, 1,3 - Diamino - 2 - phenyl - propan oder dessen im Benzolrest sulfosubstituiertes Derivat, 1,3 - oder 1,4 - Cyclohexylen - diamin, Bis - (4 - amino - cyclohex - 1 - yl) - methan, 1,8 - Di - (amino - methyl) - naphthalin, 1,4 - Di - (aminomethyl) - benzol, 1,3 - Di - (aminomethyl) - benzol, N,N' - Bis - (β - aminoethyl) - 1,4 - piperidin, 1,4- oder 1,3 - Phenylendiamin, 4 - Aminobenzylamin, 4 - Amino - phenethylamino sowie die entsprechenden N - Monoacyl - Derivate solcher Verbindungen, des weiteren Verbindungen entsprechend einer allgemeinen Formel (α), (β), (γ) und (δ).

$$H_2N-(CH_2)_2-G-(CH_2)_2-NH_2 \qquad (\alpha)$$

$$H_2N-(CH_3)_2-G-(CH_3)_2-NH_2 \qquad (\beta)$$

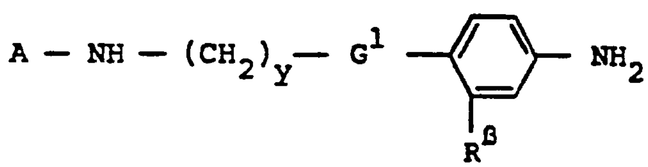

(γ)

$$H_2N-(CH_2-CH_2-NH)_v-CH_2-CH_2-NH_2 \qquad (\delta)$$

in welchen

G ein Sauerstoffatom, ein Schwafelatom, ein Sulfonyl - gruppe oder eine Gruppe der Formel —NH—, —$N(CH_3)$— oder —$N(COCH_3)$— bedeutet,

$R^{\beta}$ für ein Wasserstoffatom oder eine Sulfogruppe steht,

A die obengenannte Bedeutung besitzt,

v die Zahl 2, 3 oder 4 ist,

$G^1$ die Gruppe —NH— oder ein Sauerstoffatom ist und

y die Zahl 2 oder 3 bedeutet.

Bevorzugt sind hiervon insbesondere 1,2 - Diamino - ethan, 1,3 - Diamino - propan, 1,4 - Diamino - butan und 1,2 - Diamino - propan.

Geeignete Lösungsmittel für die Umsetzung der Halogennitro - benzole und der Aminoverbindungen sind beispielsweise Wasser, Alkanole von 1 bis 4 C-Atomen, Dioxan, Toluol, die Xylole, Chlorbenzol, o - Dichlorbenzol, m - Dichlorbenzol, Di - methylformamid und N - Methyl - pyrrolidon. Als Lösemittel kann auch das Amin selbst im Überschuß verwendet werden.

Die enhaltene Nitroverbindung der allgemeinen Formel (10)

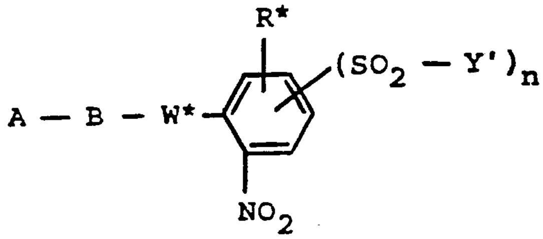

(10)

in welcher A, B, R*, W*, Y' und n die obengenannten Bedeutungen haben, kann in üblicher Weise, beispielsweise durch Kristallisation aus dieser Reaktionsmichung bzw. durch Abdestillieren des Lösemittels oder überschüssigen Amins oder durch Ansäuern und Filtration, isoliert werden. Die anschließende Reduktion der Nitrogruppe in der Nitroverbindung (10) zur Aminogruppe erfolgt in an und für sich bekannter Weise, so durch katalytische Hydrierung mit Wasserstoff an Palladium, Platin oder Raney-Nickel bie einer Temperatur zwischen 50 und 110°C und bei erhöhtem Druck oder durch Reduktion nach Béchamp mit Eisen in saurem Medium, beispielsweise mit Eisen in Ethanol/Eisessig, durchgeführt werden. Die Reduktion kann in einem hierfür geeigneten Lösemittel, wie Wasser, Methanol oder Ethanol oder einer Mischung derselben, erfolgen.

Die im Hydrieransatz enthaltene Aminoverbindung kann — nach vorheriger Abtrennung von Katalysatoren oder metallischen Reduktionsmitteln — direkt, ohne Zwischenisolierung, unter gleichzeitigem Ringschluß zum Triazol diazotiert werden. Die Diazotierung erfolgt in an und für sich üblicher Verfahrensweise, so beispielsweise mit Natriumnitrit in saltzsaurem Medium bei einer Temperatur zwischen −5°C und +15°C. Eine in der erhaltenen Benztriazolverbindung eventuell vorhandene Acylaminogruppe kann in üblicher Verfahrensweise, so beispielsweise in wäßrigem Medium bei einem pH-Wert von größer als 12 und einer Temperatur von 90 bis 100°C, zur Aminogruppe und somit zur Verbindung der allgemeinen Formel (7) hydrolysiert werden.

Die Abscheidung und Isolierung der erfindungsgemäß hergestellten Verbindungen der allgemeinen Formel (1) aus den Syntheselösungen kann nach allgemein bekannten Methoden erfolgen, so beispielsweise entweder durch Ausfällen aus dem Reaktionsmedium mittels Elektrolyten, wie beispielsweise Natriumchlorid oder Kaliumchlorid, oder durch Eindampfen der Reaktionslösung, beispielsweise Sprühtrocknung, wobei dieser Reaktionslösung eine Puffersubstanz zugefügt werden kann.

Die erfindungsgemäßen Verbindungen der allgemeinen Formel (1) haben faserreaktive Eigenschaften und besitzen wertvolle Farbstoffeigenschaften. Sie können deshalb zum Färben (einschließlich Bedrucken) von natürlichen, regenerierten oder synthetischen hydroxygruppenhaltigen und/oder carbonamidgruppenhaltigen Materialien, beispielsweise in Form von Flächengebilden, wie Papier und Leder, oder in der Masse, von Polyamid oder Polyurethan, insbesondere aber von solchen Materialien in Faserform, wie Cellulosefasermaterialien, Seide, Wolle und synthetische Polyamid- und Polyurethanfasern, verwendet werden. Auch können die bei der Synthese der erfindungsgemäßen Verbindungen anfallenden Lösungen, gegebenenfalls nach Zusatz einer Puffersubstanz, gegebenenfalls auch nach Konzentrierung, direkt als Flüssigpräparation der färberischen Verwendung zugeführt werden.

Die erfindungsgemäßen Verbindungen der Formel (1) lassen sich, gemäß der erfindungsgemäßen Anwendung, auf den genannten Substraten, insbesondere auf den genannten Fasermaterialien, nach den für wasserlösliche, insbesondere faserreaktive, Farbstoffe bekannten Anwendungstechniken applizieren und fixieren, so beispielsweise, indem man die Dioxazinverbindung der allgemeinen Formel (1) in gelöster Form auf das Substrat aufbringt oder sie darin einbringt und sie auf diesem oder in diesem, gegebenenfalls durch Hitzeeinwirkung und/oder gegebenenfalls durch Einwirkung eines alkalisch wirkenden Mittels, fixiert. Solche Färbe- und Fixierweisen sind in der Literatur zahlreich beschrieben.

Gegenstand der vorliegenden Erfindung ist deshalb auch die Verwendung der erfindungsgemäßen Verbindungen der allgemeinen Formel (1) zum Färben (einschließlich Bedrucken) von hydroxy- und/oder carbonamidgruppenhaltigen Materialien bzw. Verfahren zu deren Anwendung auf diesen Substraten.

Bevorzugt kommen die Materialien in Form von Fasermaterialien zur Anwendung, insbesondere in Form von Textilfasern, wie Garnen, Wickelkörpern und Geweben. Hierbei kann man analog bekannten Verfahrensweisen der Applikation und Fixierung von faserreaktiven Farbstoffen vorgehen.

Die mit erfindungsgemäßen Verbindungen der allgemeinen Formel (1) hergstellten Färbungen und Drucke zeichnen sich durch reine, vorwiegend blaue Farbtöne aus. Insbesondere die Färbungen und Drucke auf Cellulosefasermateralien bedsitzen sehr hohe Farbstärken und ebenso sehr gute Lichtechtheiten, einschließlich guter Naßlicht- und Schweißlichtechtheiten, ebenso gute Hypochloritbleich- und Chlorbadewasserechtheiten, weiterhin vorzügliche Naßechtheiten wie beispielsweise gute bis sehr gute Waschechtheiten bei 60 bis 95°C, auch in Gegenwart von Perboraten, saure und alkalische Walk-, Überfärbe- und Schweißechtheiten, Alkali-, Säure-, Wasser- und Seewasserechtheiten, desweiteren eine gute Plissierechtheit, Bügelechtheit und Reibechtheit. Ebenso besitzen sie eine gute Naßliegeechtheit und eine sehr gute Säurelagerbeständigkeit ("acid fading") beim Lagern von feuchten, noch Essigsäure enthaltendem, gefärbtem Material. Deswieteren sind die Färbungen gegen die üblichen Kunstharzappreturen stabil. Ein Teil der erfindungsgemäßen Verbindungen (Farbstoffe) sind in der Reinheit des Farbtones und in wichtigen Echtheitseigenschaften mit faserreaktiven Anthrachinonfarbstoffen vergleichbar.

Die nachstehenden Beispiele dienen zur Erläuterung der Erfindung. Die Teile sind Gewichsteile, die Prozentangaben stellen Gewichtsprozente dar, sofern nicht anders vermerkt. Gewichsteile beziehen sich zu Volumenteilen wie Kilogramm zu Liter.

Beispiel 1

a) 492 Teile 3 - (β - Sulfoethylsulfonyl) - 4 - [4' - β - (β - hydroxyethylsulfonyl - phenyl) - ethylamino] - anilin werden in 2000 Teilen Wasser bei 60°C klar gelöst. 124 Teile Chloranil werden eingetragen, und es wird während der Reaktion mit etwa 90 Teilen Natriumbicarbonat ein pH-Wert von 6 bis 6,5 bei einer Reaktionstemperatur von etwa 65°C gehalten. Der Ansatz wird noch eine Stunde nachgerührt, das Reaktionsprodukt bei etwa 30°C abgesaugt, mit 400 Teilen Wasser gewaschen und unter reduziertem Druck bei 70°C getrocknet.

b) 116 Teile des unter a) erhaltenen Produktes werden bei einer Temperatur zwischen 20 und 25°C in 600 Teile 15%iges Oleum eingetragen. Der Reaktionsansatz wird anschließend noch etwa drei Stunden bei dieser Temperatur nachgerührt; sodann werden 48 Teile Natrium-peroxodisulfat eingetragen, und zwar in der Weise, daß die Reaktionstemperatur bei 20 bis 25°C gehalten werden kann. Man rührt noch 10 Stunden bei dieser Temperatur nach, läßt den Ansatz sodann auf Eis laufen, filtriert die ausgefallene erfindungsgemäße Verbindung ab, löst sie wieder in etwa 1000 Teilen Wasser, stellt mit Natriumcarbonat einen pH-Wert von 5 ein und salzt sie gegebenenfalls nach vorheriger üblicher Klärung der Lösung, mit Natriumchlorid aus.

Die erfindungsgemäße Triphendioxazin-Verbindung kann auch in Form ihres Natriumsalzes durch Eindampfen oder Sprühtrocknen des vereinigten Filtrates erhalten werden. Sie besitzt, in Form der freien Säure geschrieben, die mutmäßliche Formel

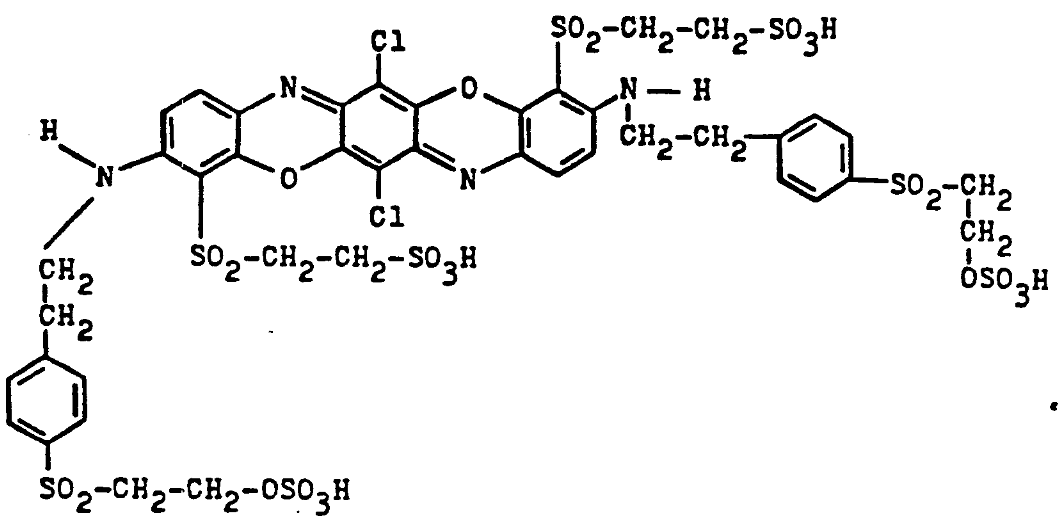

(die jeweilige β - Sulfoethylsulfonyl - Gruppe kann auch in der anderen ortho - Stellung zur (β - Sulfatoethylsulfonyl) - phenyl - ethylamino - Gruppe gebunden sein, befindet sich jedoch mit größerer Wahrscheinlichkeit in der in der obigen Formel angegebenen Stellung).

Diese erfindungsgemäße Verbindung besitzt sehr gute faserreaktive Farbstoffeigenschaften. Sie färbt die in der Beschreibung genannten Materialien, insbesondere Cellulosefasermaterialien, wie Baumwolle, nach den in der Technik üblichen und bekannten Verfahrensweisen der Applikation und Fixierung von faserreaktiven Farbstoffen in farbstarken, reinen rotstichig blauen Tönen (entsprechend der Farbkennzahl

13 von Colour Index Hue Indication Chart) mit guten Echtheiten, wie insbesondere guter Lichtechtheit der trockenen oder feuchten, wie mit Trinkwasser befeuchteten Färbung, guter alkalischen Schweißlichtechtheit, Chlorbadewasserechtheit, Hypochloritechtheit, alkalischer Schweißechtheit, Waschechtheit, auch in Gegenwart von Perboraten, Naßliegeechtheit und Säurelagerbeständigkeit. In wäßriger Lösung zeigt sie im sichtbaren Bereich ein Absorptionsmaximum bei 614 nm.

c) Die unter a) eingesetzte Anilinverbindung kann beispielsweise wie folgt hergestellt werden: 330 Teile 3 - (β - Sulfoethylsulfonyl) - 4 - chlor - nitrobenzol werden in einer wäßrigen Natronlauge aus 48 Teilen Natriumhydroxid und 2600 Teilen Wasser suspendiert; 320 Teile 2 - [4' - (β - Hydroxyethylsulfonyl) - phenyl] - ethyl - amin - hydrochlorid werden sofort unter Rühren eingetragen und die Reaktionstemperatur auf 70°C erhöht; während der Umsetzung wird der pH-Wert bei 10 mittels einer wäßrigen Natronlauge gehalten. Nach Beendigung der Umsetzung wird der Ansatz heiß geklärt und mit verdünnter Salzsäure auf einen pH-Wert von 6 bis 7 eingestellt. Aus dem wäßrigen Medium kristallisiert beim Abkühlen das 2 - (β - Sulfoethylsulfonyl) - 4 - nitro - 1 - [4' - (β - hydroxyethylsulfonyl) - phenyl] - ethylamino - benzol in hoher Reinheit aus.

Es zeigt folgende Daten in der 1H-NMR-Analyse (in $D_6$-Dimethylsulfoxid mit Tetramethylsilan als inneren Standard): $\delta$ = 2,61 ppm (t, 2H); 3,05 ppm (t, 2H); 3,43 ppm (t, 2H); 3,46 ppm (t, 2H); 3,63 ppm (m, 2H); 3,70 ppm (m, 2H); 4,82 ppm (t, 0H); 7,11 ppm (d, 1H); 7,27 ppm (t, NH); 7,68 ppm (d, 2H); 7,84 ppm (d, 2H); 8,24 ppm (dd, 1H); 8,36 ppm (d, 1H).

Diese Nitroverbindung wird durch katalytische Hydrierung zur Anilinverbindung reduziert, indem man 261 Teile der Nitroverbindung in 1000 Teilen Wasser löst und in Gegenwart eines Pd/C-Katalysators in einem Autoklaven bei einer Temperatur bis 100°C und einem Wasserstoffdruck von 50 bar hydriert. Der Katalysator wird anschließend abfiltriert, das Filtrat angesäuert und abgekühlt. Aus ihm kristallisiert die Anilinverbindung in guter Ausbeute und hoher Reinheit aus.

Beispiel 2

a) 145 Teile 3 - (β - Sulfo - ethylsulfonyl) - 4 - [3 - (β - hydroxyethylsulfonyl) - 4 - methoxy - phenylamino] - anilin werden in 600 Teilen Wasser bei 60°C klar gelöst. 37 Teile Chloranil werden eingetragen, und es wird während der Reaktion mit etwa 30 Teilen Natriumbicarbonat ein pH-Wert von 6 bis 6,5 bei einer Reaktionstemperatur von etwa 65°C gehalten. Der Ansatz wird noch eine Stunde nachgerührt, das Reaktionsprodukt bei etwa 20°C abgesaugt, mit 50 Teilen Ethanol gewaschen und unter reduziertem Druck bei 60°C getrocknet.

b) 117 Teile des unter a) erhaltenen Produktes werden bei einer Temperatur zwischen 20 und 25°C in 1000 Teile 10%iges Oleum eingetragen. Der Reaktionsansatz wird anschließend noch etwa drei Stunden bei dieser Temperatur nachgerührt; sodann werden 47 Teile Natrium-peroxodisulfat eingetragen, und zwar in der Weise, daß die Reaktionstemperatur bei 20 bis 25°C gehalten werden kann. Man rührt noch 10 Stunden bei dieser Temperatur nach, läßt den Ansatz sodann auf Eis laufen, stellt ihn mit Calciumcarbonat auf einen pH-Wert zwischen 1 und 1,5, sodann mit Natriumcarbonat auf einen pH-Wert von 5,5, filtriert das ausgefallene Calciumsulfat ab, wäscht es mit Wasser aus und vereinigt die Filtrate.

Die erfindungsgemäße Triphendioxazin-Verbindung kann in Form ihres Natriumsalzes durch Eindampfen oder Sprühtrocknen des vereinigten Filtrates erhalten werden. Sie besitzt, in Form der freien Säure geschrieben, die mutmäßliche Formel

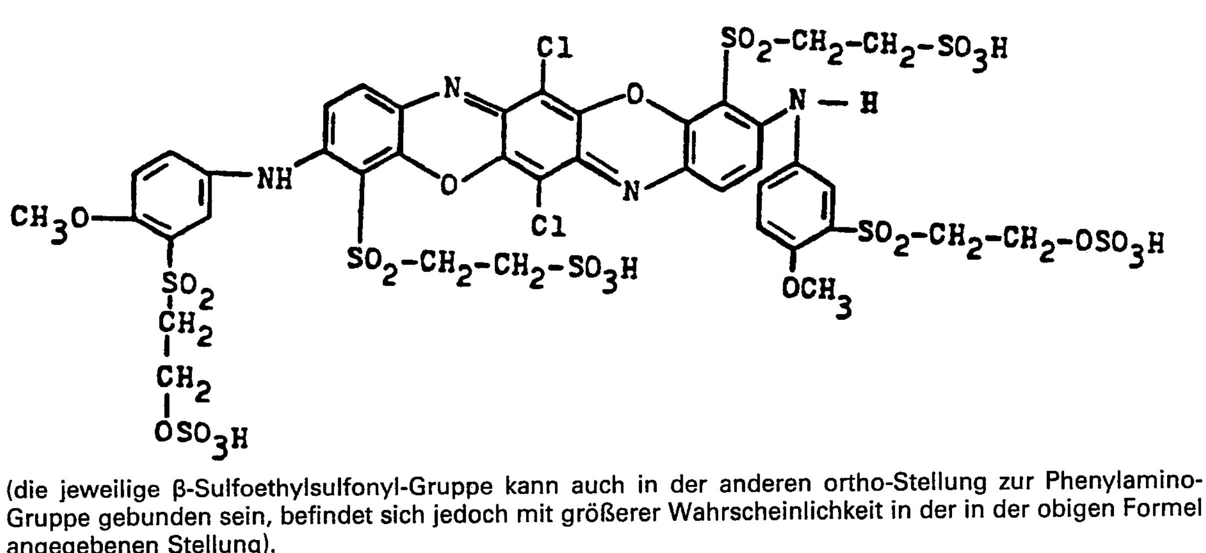

(die jeweilige β-Sulfoethylsulfonyl-Gruppe kann auch in der anderen ortho-Stellung zur Phenylamino-Gruppe gebunden sein, befindet sich jedoch mit größerer Wahrscheinlichkeit in der in der obigen Formel angegebenen Stellung).

Diese erfindungsgemäße Verbindung besitzt sehr gute faserreaktive Farbstoffeigenschaften. Sie färbt die in der Beschreibung genannten Materialien, insbesondere Cellulosefasermaterialien, wie Baumwolle, nach den in der Technik üblichen und bekannten Verfahrensweisen der Applikation und Fixierung von faserreaktiven Farbstoffen in farbstarken, reinen rotstichig blauen Tönen (Farbkennzahl 13) mit guten

Echtheiten, wie insbesondere guter Lichtechtheit der trockenen oder feuchten, wie mit Trinkwasser befeuchteten Färbung, guter alkalischen Schweißlichtechtheit, Chlorbadewasserechtheit, Hypochloritechtheit, alkalischer, Schweißechtheit, Waschechtheit, auch in Gegenwart von Perboraten, Naßliegeechtheit und Säurelagerbeständigkeit.

In wäßriger Lösung zeigt sie im sichtbaren Bereich ein Absorptionsmaximum bei 583 nm.

c) Die unter a) eingesetzte Anilinverbindung kann beispielsweise wie folgt hergestellt werden: 165 Teile 3 - (β - Sulfoethylsulfonyl) - 4 - chlor - nitrobenzol und 116 Teile 3 - (β - Hydroxyethylsulfonyl) - 4 - methoxy - anilin werden in 1000 Teilen Wasser und in Anwesenheit von 14 Teilen Magnesiumoxid 15 Stunden bei 100°C erhitzt. Der Ansatzt wird nach dem Erkalten geklärt, mit konzentrierter Salzsäure angesäuert; das synthetsierte 3 - (β - Sulfoethylsulfonyl) - 4 - [3 - (β - hydroxyethylsulfonyl) - 4 - methoxy - phenylamino] - nitrobenzol wird anschließend in hoher Reinheit mittels 150 Teilen Natriumchlorid ausgesalzen und isoliert.

$^1$H-NMR-Analyse (in $D_2O$): $\delta$ = 3,15—3,42 ppm (m, 2H); 3,55—4,15 ppm (m, 9H); 6,88 ppm (d, 1H); 7,33 ppm (d, 1H); 7,68 ppm (dd, 1H); 7,8 ppm (d, 1H); 8.08 ppm (dd, 1H); 8,55 ppm (d, 1H); die Protonen der Hydroxy-, Amino- und Sulfogruppen waren nicht sichtbar.

Diese Nitroverbindung läßt sich anschließend durch Hydrierung in Anwesenheit eines Nickelkatalysators, beispielsweise in Methanol bei einer Temperatur von etwa 100°C und einem Druck von 50 bar Wasserstoff, in die entsprechende Anilinverbindung überführen.

Beispiel 3

a) 523 Teile 3 - (β - Sulfoethylsulfonyl) - 4 - [3 - (β - hydroxyethylsulfonyl) - 4 - (β - hydroxyethylamino) - phenylamino] - anilin werden in 2000 Teilen Wasser bei 65°C klar gelöst. 124 Teile Chloranil werden eingetragen, und es wird während der Reaktion mit etwa 80 Teilen Natriumbicarbonat ein pH-Wert von 6 bis 6,5 bei einer Reaktionstemperatur von etwa 65°C gehalten. Der Ansatz wird noch drei Stunden nachgerührt, das Reaktionsprodukt nach Abkühlen abgesaugt, mit 200 Teilen Wasser gewaschen und unter reduziertem Druck bei 70°C getrocknet.

b) 122 Teile des Produktes aus a) werden bei einer Temperatur zwischen 20 und 25°C in 500 Teile 20%iges Oleum eingetragen. Der Reaktionsansatz wird anschließend noch etwa 15 Stunden bei dieser Temperatur nachgerührt; sodann werden 48 Teile Natrium-peroxodisulfat eingetragen, und zwar in der Weise, daß die Reaktionstemperatur bei 20 bis 25°C gehalten werden kann. Man rührt noch 10 Stunden bei dieser Temperatur nach, läßt den Ansatz sodann auf Eis laufen, stellt ihn mit Calciumcarbonat auf einen pH-Wert zwischen 1 und 1,5, sodann mit Natriumcarnbonat auf einen pH-Wert von 6, filtriert das ausgefallene Calciumsulfat ab, wäscht es mit Wasser aus und vereinigt die Filtrate. Noch in Lösung befindliche Calciumionen können mit Oxalsäure gefällt werden.

Die erfindungsgemäße Triphendioxazin-Verbindung kann in Form ihres Natriumsalzes durch Eindampfen oder Sprühtrocknen des vereinigten Filtrates erhalten oder Aussalzen mittels Elektrolytsalzen, wie Natrium- und Kaliumchlorid, isoliert werden. Sie besitzt, in Form der freien Säure geschrieben, die mutmäßliche Formel

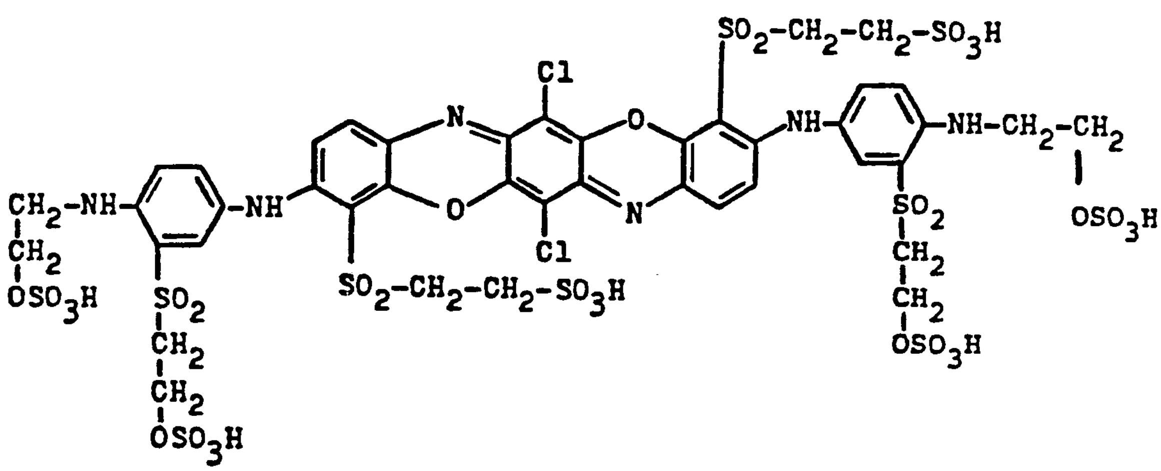

(die jeweilige β-Sulfoethylsulfonyl-Gruppe kann auch in der anderen ortho-Stellung zur Phenylamino-Gruppe gebunden sein, befindet sich jedoch mit größerer Wahrscheinlichkeit in der in der obigen Formel angegebenen Stellung).

Diese erfindungsgemäße Verbindung besitzt sehr gute faserreaktive Farbstoffeigenschaften. Sie färbt die in der Beschreibung genannten Materialien, insbesondere Cellulosefasermaterialien, wie Baumwolle, nach den in der Technik üblichen und bekannten Verfahrensweisen der Applikation und Fixierung von faserreaktiven Farbstoffen in farbstarken, reinen grünstichig blauen Tönen (Farbkennzahl 14) mit guten Echtheiten, wie insbesondere guter Lichtechtheit der trockenen oder feuchten, wie mit Trinkwasser

befeuchteten Färbung, guter alkalischen Schweißlichtechtheit, Chlorbadewasserechtheit, Hypochloritechtheit, alkalischer, Schweißechtheit, Waschechtheit, auch in Gegenwart von Perboraten, Naßliegeechtheit und Säurelagerbeständigkeit.

In wäßriger Lösung zeigt sie im sichtbaren Bereich ein Absorptionsmaximum bei 620 nm.

c) Die unter a) eingesetzte Anilinverbindung kann analog dem Beispel 2c) hergestellt werden, wobei man als Anilinverbindung 131 Teile 3 - (β - Hydroxyethylsulfonyl) - 4 - (β - hydroxyethylamino) - anilin einsetzt. Nach erfolgter Umsetzung und Klärung des Ansatzes bei einem pH-Wert von 6 erfolgt die Hydrierung direkt in der Lösung.

Beispiele 4 bis 30

In den nachfolgenden Tabellenbeispielen sind weitere erfindungsgemäße Triphendioxazinverbindungen entsprechend der allgemeinen Formel (1) mit Hilfe ihrer Formelreste beschrieben (wobei W identisch mit $W^1$ ist und R für ein Wasserstoffatom steht). Sie lassen sich in erfindungsgemäßer Weise herstellen, beispielsweise analog den obigen Ausführungsbeispielen durch Umsetzung einer aus dem jeweiligen Tabellenbeispiel ersichtlichen 1,4-Benzochinon-Verbindung entsprechend der allgemeinen Formel (5) mit einer Verbindung der allgemeinen Formel (4), in welcher R für ein Wasserstoffatom steht und B, W, T und n die aus dem jeweiligen Tabellenbeispiel ersichtlichen Bedeutungen haben, wobei anstelle der für Y in der Tabellenangabe stehenden ß-Sulfatoethyl-Gruppe in der Verbindung (4) die ß-Hydroxyethyl-Gruppe für den Formelrest Y' steht, und durch nachträgliche Sulfatierung und Cyclisierung. Diese erfindungsgemäßen Triphendioxazin-Verbindungen besitzen ebenfalls sehr gute faserreaktive Farbstoffeigenschaften und liefern auf den in der Beschreibung ganannten Materialien, wie insbesondere Cellulosefasermaterialien, farbstarke, echte Färbungen mit dem in dem jeweiligen Tabellenbeispiel auf Baumwolle angegebenen Farbton (mit der in Klammern angegebenen Farbkennzahl nach Colour Index Hue Indication Chart).

| Bsp. | Rest —B—W—($SO_2$—Y)$_n$ | Verbindung der Formel (1) mit . . . | | | Verbindung (5) = | Farbton |
|---|---|---|---|---|---|---|
| | | T ist . . . | $X^1$ ist . . . | $X^2$ ist . . . | . . .-1,4-benzochinon | |
| 4 | β-(4-β'-Sulfatoethylsulfonyl-phenyl)ethylamino | β-Sulfoethyl | Brom | Brom | 2,3,5,6-Tetra-bromo-. . . | rotstichig blau (13) |
| 5 | " | " | Methyl | Methyl | 2,5-Dimethyl-3,6-dichloro-. . . | " |
| 6 | " | " | Methyl | Chlor | 2-Methyl-3,5,6-trichloro-. . . | " |
| 7 | 3-(β-Sulfatoethylsulfonyl)-4-methoxyphenylamino | " | Brom | Brom | 2,3,5,6-Tetra-bromo-. . . | " |
| 8 | " | " | Methyl | Methyl | 2,5-Dimethyl-3,6-dichloro-. . . | " |
| 9 | " | " | Methyl | Chlor | 2-Methyl-3,5,6-trichloro-. . . | " |
| 10 | 3-(β-Sulfatoethylsulfonyl)-4-(β-sulfato-ethylamino)-phenylamino | " | Brom | Brom | 2,3,5,6-Tetra-bromo-. . . | grünstichig blau (14) |
| 11 | " | " | Methyl | Methyl | 2,5-Dimethyl-3,6-dichloro-. . . | " |
| 12 | " | " | Methyl | Chlor | 2-Methyl-3,5,6-trichloro-. . . | " |
| 13 | 3-(β-Sulfatoethylsulfonyl)-4-(β-sulfoethylamino)-phenylamino | " | Brom | Brom | 2,3,5,6-Tetra-bromo-. . . | " |
| 14 | 3-(β-Sulfatoethylsulfonyl)-4-(N-methyl-N-β-sulfo-ethyl)-amino-phenylamino | " | Chlor | Chlor | 2,3,5,6-Tetra-chloro-. . . | " |
| 15 | 3-(β-Sulfatoethylsulfonyl)-4-ethylamino-phenylamino | " | " | " | " | " |
| 16 | 4-(β-Sulfatoethylsulfonyl)-benzylamino | " | " | " | " | rotstichig blau (13) |
| 17 | β-(4-β'-Sulfatoethylsulfonyl-phenoxy)-ethylamino | " | " | " | " | " |

| Bsp. | Rest —B—W—($SO_2$—Y)$_n$ | Verbindung der Formel (1) mit . . . T ist . . . | X$^1$ ist . . . | X$^2$ ist . . . | Verbindung (5) = . . .-1,4-benzochinon | Farbton |
|---|---|---|---|---|---|---|
| 18 | γ-N-(2-Amino-4-β'-sulfatoethylsulfonyl) -phenylamino)-n-propyl-amino | " | " | " | " | " |
| 19 | β-(β'-Sulfatoethylsulfonyl)-ethylamino | " | " | " | " | " |
| 20 | γ-(β'-Sulfatoethylsulfonyl)-n-propylamino | " | " | " | " | " |
| 21 | 3-(β-Sulfatoethylsulfonyl)-phenylamino | " | " | " | " | " |
| 22 | 6,8-Di-(β-Sulfatoethylsulfonyl)-naphth-2'-yl-amino | " | " | " | " | " |
| 23 | 6-(β-Sulfatoethylsulfonyl)-8-sulfonaphth-2'-yl-amino | " | " | " | " | " |
| 24 | 3-(β-Sulfatoethylsulfonyl)-4-methylanilino | " | " | " | " | " |
| 25 | 4-(β-Sulfatoethylsulfonyl)-phenylamino | " | " | " | " | " |
| 26 | 3-(β-Sulfatoethylsulfonyl)-4-(γ-sulfato-n-propyl-amino)-phenylamino | " | " | " | " | grünstichig blau )14) |
| 27 | β-(4-β'-Sulfatoethylsulfonyl-phenyl)-ethylamino | " | Fluor | Fluor | 2,3,5,6-Tetra-fluoro-. . . | rotstichig blau )13) |
| 28 | " | β-Carboxy-ethyl | Chlor | Chlor | 2,3,5,6-Tetra-chloro-. . . | " |
| 29 | " | β-Sulfo-n-propyl | " | " | " | " |
| 30 | " | Carboxy-methyl | " | " | " | " |

Beispiel 31

a) 547 Teile der Verbindung der Formel

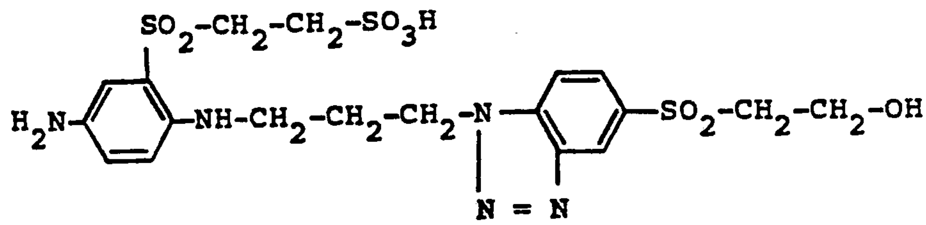

werden in 2000 Teilen Wasser bei pH 6 und 60°C gelöst. 124 Teile Chloranil werden eingetragen, und es wird während der Reaktion mit etwa 90 Teilen Natriumbicarbonat ein pH-Wert von 6 bis 6,5 bei einer Reaktionstemperatur von etwa 65°C gehalten. Der Ansatz wird noch 4 Stunden nachgerührt, das Reaktionsprodukt bei etwa 30°C abgesaugt, mit 1000 Teilen Wasser gewaschen und unter reduziertem Druck bei 70°C getrocknet.

b) 127 Teile des unter a) erhaltenen Produktes werden bei einer Temperatur zwischen 20 und 25°C in 650 Teile 13%iges Oleum eingetragen. Der Reaktionsansatz wird anschließend noch etwa 12 Stunden bei dieser Temperatur nachgerührt, sodann werden 48 Teile Natrium-peroxodisulfat eingetragen, und zwar in der Weise, daß die Reaktionstemperatur bei 20 bis 25°C gehalten werden kann. Man rührt noch 10 Stunden bei dieser Temperatur nach, läßt den Ansatz sodann auf Eis laufen, filtriert die ausgefallene erfindungsgemäße Verbindung ab, löst sie wieder in etwa 1000 Teilen Wasser, stellt mit Natriumcarbonat einen pH-Wert von 5 ein und salzt sie, gegebenenfalls nach vorheriger üblicher Klärung der Lösung, mit Natriumchlorid aus.

Die erfindungsgemäße Triphendioxazin-Verbindung kann auch in Form ihres Natriumsalzes durch Eindampfen oder Sprühtrocknen des vereinigten Filtrates erhalten werden. Sie besitzt, in Form der freien Säure geschrieben, die mutmaßliche Formel

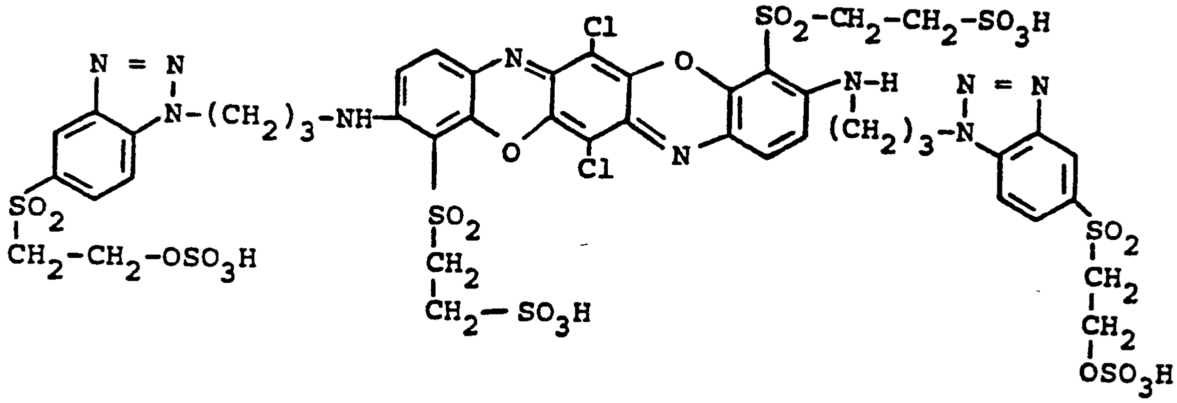

(die jeweilige β-Sulfoethylsulfonyl-Gruppe kann auch in der anderen o-Stellung zur (β-Sulfatoethylsulfonyl)-benztriazolyl-propylamino-Gruppe gebunden sein, befindet sich jedoch mit größere Wahrscheinlichkeit in der in der obigen Formel angegebenen Stellung).

Diese erfindungsgemäße Verbindung besitzt sehr gute faserreaktive Farbstoffeigenschaften. Sie färbt die in der Beschreibung genannten Materialien, insbesondere Cellulosefasermaterialien, wie Baumwolle, nach den in der Technik üblichen und bekannten Verfahrensweisen der Applikation und Fixierung von faserreaktiven Farbstoffen in farbstarken, reinen rotstichig blauen Tönen (entsprechend der Farbkennzahl 13 von Col. Index Hue Indication Chart) mit guten Echtheiten, wie insbesondere guter Lichtechtheit der trockenen oder feuchten, wie mit Trinkwasser befeuchteten Färbung, guter alkalischer Schweißlichtechtheit, Chlorbadewasserechtheit, Hypochloritechtheit, alkalischer Schweißechtheit, Waschechtheit, auch in Gegenwart von Perboraten, Naßliegeechtheiten und Säurelagerbeständigkeit. In wäßriger Lösung zeigt sie im sichtbaren Bereich ein Absorptionsmaximum bei 617 nm.

c) Die unter a) eingesetzte Anilinverbindung kann beispielsweise wie folgt hergestellt werden: 352 Teile des Natriumsalzes von 2-(β-Sulfoethylsulfonyl)-4-nitro-chlorbenzol werden zu einer auf 40°C vorgeheizten Mischung von 270 Teilen γ-[5-(β-hydroxyethylsulfonyl)-benzotriazol-1-yl]-n-propylamin in 1000 Teilen Wasser und 550 Teilen Triethanolamin gegeben. Innerhalb 3 Stunden wird die Mischung auf 100 bis 110°C aufgeheizt, wobei das Wasser teilweise abdestilliert. Man rührt weitere 5 Stunden bei 115 bis 120°C zur quantitativen Umsetzung nach, gibt bei 100°C 2000 Teile Wasser zu und klärt die Lösung bei 80

bis 90°C. Aus dem wäßrigen Medium kristallisiert beim Abkühlen und nach Zugabe von NaCl das Natriumsalz der Verbindung der Formel

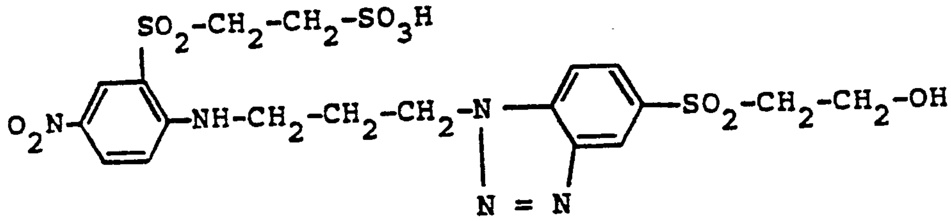

in hoher Ausbeute und Reinheit aus.
Es zeigt folgende Daten in der 1H-NMR-Analyse (in $D_6$-DMSO mit TMS als innerem Standard): δ = 2,3 ppm (m, 2H); 2,7 ppm (m, 2H); 3,5 ppm (m, 2H); 3,6 ppm (m, 4H); 3,7 ppm (m, 2H); 4,9 ppm (t, 2H); 4,95 ppm (t, 0H); 7,1 ppm (d, 1H);7,37 ppm (t, NH); 8,03 ppm (dd, 1H); 8,22 (d, 1H); 8,25 ppm (dd, 1H); 8,38 ppm (d, 1H); 8,62 ppm (s, 1H).

Diese Nitroverbindung wird durch katalytische Hydrierung zur Anilinverbindung reduziert, indem man 289 Teile der Nitroverbindung in 1000 Teilen Wasser löst und in Gegenwart eines Pd/C-Katalysators in einem Autoklaven bei einer Temperatur bis 100°C und einem Wasserstoffdruck von 50 bar hydriert. Der Katalysator wird anschließend abfiltriert.

Das Hydrierungsprodukt kann direkt zur Umsetzung nach a) verwendet werden.

d) Die unter c) eingesetzte 5-(β-Hydroxyethylsulfonyl)-benztriazolyl-propylamino-Verbindung kann beispielsweise wie folgt hergestellt werden: 260 Teile γ-[4-(β-Hydroxyethylsulfonyl)-2-amino-phenylamino)]-n-propylamin werden in etwa 1800 Teilen einer salzsauren wäßrigen Lösung bei 0 bis 5°C mittels einer wäßrigen Natriumnitritlösung in üblicher Weise diazotiert. Der Ringschluß erfolgt sofort und quantitativ. Die Lösung kann direkt in c) eingesetzt werden. Eine isolierte Probe zeigt folgende Daten in der 1H-NMR-Analyse (in $D_6$-DMSO mit TMS als inneren Standard): δ = 2,24 ppm (m,2 H); 2,85 ppm (m, 2H); 3,6 ppm (m, 4H); 4,92 ppm (t, 2H); 8,04 ppm (dd, 1H); 8,27 ppm (d, 1H); 8,6 ppm (s, 1H); mobile Protonen (OH, $NH_2$)

e) Die unter d) eingesetzte 4-(β-Hydroxyethylsulfonyl)-2-amino-phenylaminopropylamin-Verbindung kann beispielsweise wie folgt erhalten werden: 530 Teile 4-(β-Hydroxyethylsulfonyl)-2-nitro-chlorbenzol werden bei 70 bis 80°C langsam in 750 Teile n-Propylendiamin eingetragen. Nach quantitativer Umsetzung wird der Ansatz erforderlichenfalls auf Wasser ausgerührt und das Produkt abgesaugt. Man erhält die Nitroverbindung in guter Ausbeute und hoher Reinheit mit einem Fp. von 110 bis 112°C. Sie zeigt folgende Daten in der 1H-NMR-Analyse (im $D_6$-DMSO mit TMS als inneren Standard): δ = 1,7 ppm (m, 2H); 2,66 ppm (t, 2H); 3,5 ppm (m, 4H); 3,68 ppm (m, 2H); 7,25 ppm (d, 1H); 7,9 ppm (dd, 1H); 8,5 ppm (d, 1H); mobile Protonen ($NH_2$, OH).

Diese Nitroverbindung wird durch katalytische Hydrierung zur Anilinverbindung reduziert, indem man 303 Teile der Nitroverbindung in 1200 Teilen Wasser in Gegenwart eines Pd/C-Katalysators in einem Autoklaven bei einer Temperatur bis 100°C und einem Wasserstoffdruck von 50 bar hydriert. Der Katalysator wird anschließend abfiltriert; das Filtrat kann direkt in d) weiterverarbeitet werden. Eine isolierte Probe zeigt folgende Daten in der 1H-NMR-Analyse (in $D_6$-DMSO mit TMS als inneren Standard): δ = 1,9 ppm (m, 2H); 2,88 ppm (t, 2H); 3,2 ppm (m, 4H); 3,58 ppm (m, 2H); 6,51 ppm (dd, 1H); 6,92 ppm (d, 1H); 6,96 ppm (dd, 1H); mobile Protonen bei 4,9 ppm (OH), 5,2 ppm ($NH_2$), 5,74 ppm (NH) und 8,2 ppm ($NH_2$).

f) Die unter c) eingesetzte Chlornitroverbindung kann beispielsweise wie folgt erhalten werden: 260 Teile 2-(β-Hydroxyethylsulfonyl)-4-nitro-chlorbenzol werden in 800 teilen 100%iger Schwefelsäure und 400 Teilen 20%igem Oleum quantitativ in den Schwefelsäureester überführt, der sodann durch Ausrühren auf Eis/Kochsalz ausgefällt, unverzüglich abgesaugt und mit 20%iger wäßriger NaCl-Lösung säurefrei gewaschen (Ausbeute nahezu quantitativ) und anschließend in 1500 Teilen Wasser bei 50°C gelöst wird. Eine Lösung von 125 Teilen $Na_2SO_3$ in 400 Teilen Wasser wird schnell zugegeben. Nach kurzer Mischungszeit erfolgt spontan eine Fällung. Nach 1-stündigem Nachrühren bei 50°C fällt der pH auf ca. 4. Man saugt unterhalb 10°C ab und trocknet das Produkt gegebenenfalls bei 80°C unter reduziertem Druck. Es zeigt folgende Daten in der 1H-NMR-Analyse (in $D_6$-DMSO mit TMS als inneren Standard): δ = 2,7 ppm (m, 2H); 3,8 ppm (m, 2H); 8,06 ppm (dd, 1H); 8,55 ppm (dd, 1H); 8,64 ppm (d, 1H).

Beispiele 32 bis 70

In den nachfolgenden Tabellenbeispielen sind weitere erfindungsgemäße Triphendioxazinverbindungen entsprechend einer allgemeinen Formel (1A)

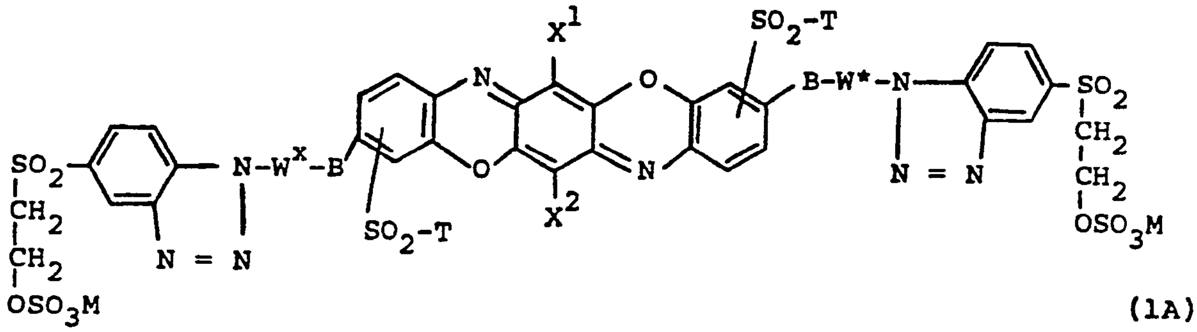

(1A)

(worin M die in der Beschreibung angegebene Bedeutung hat) mit Hilfe ihrer Formelreste beschrieben (hierin stellt der Rest $W^x$ jeweils die "spiegelbildliche" Gruppe des aufgezeigten Restes W* dar). Sie lassen sich in erfindungsgemäßer Weise, beispielsweise analog den obigen Ausführungsbeispielen durch Umsetzung einer aus dem jeweiligen Tabellenbeispiel ersichtlichen 1,4-Benzochinon-Verbindung entsprechend der allgemeinen Formel (5) mit einer Verbindung der allgemeinen Formel (4A)

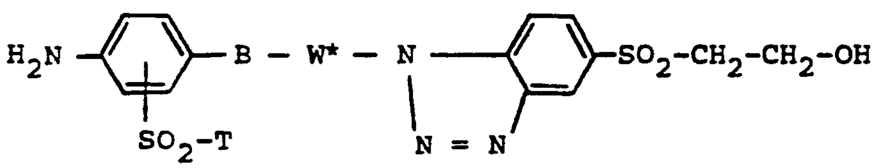

(4A)

und nachträglicher Sulfatierung und Cyclisierung, herstellen. Diese erfindungsgemäßen Triphendioxazin-Verbindungen besitzen ebenfalls sehr gute faserreaktive Farbstoffeigenschaften und liefern auf den in der Beschriebung genannten Materialien, wie insbesondere Cellulosefasermaterialien, farbstarke, echte Färbungen mit dem in dem jeweiligen Tabellenbeispiel auf Baumwolle angegebenen Farbton (mit der in Klammern angegebenen Farbkennzahl nach Colour Index Hue Indication Chart).

| Bsp. | Verbindung entsprechend Formel (1A) | | | | | Verbindung (5) = ... 1,4-benzochinon | Farbton |
|---|---|---|---|---|---|---|---|
| | Rest B | Rest T | Rest W* | $X^1$ ist ... | $X^2$ ist ... | | |
| 32 | —NH— | β-Sulfoethyl | 1,2-Ethylen | Brom | Brom | 2,3,5,6-Tetrabromo-... | rotstichig blau (13) |
| 33 | —NH— | " | " | Methyl | Methyl | 2,5-Dimethyl-3,6-dichloro-... | " |
| 34 | —NH— | " | " | Methyl | Chlor | 2-Methyl-3,5,6-trichloro-... | " |
| 35 | —NH— | " | " | Methoxy | Methoxy | 2,3,5,6-Tetramethoxy-... | " |
| 36 | —NH— | " | " | Chlor | Chlor | 2,3,5,6-Tetrachloro-... | " |
| 37 | —NH— | " | 1,3-Propylen | Brom | Brom | 2,3,5,6-Tetrabromo-... | " |
| 38 | —NH— | " | " | Methyl | Methyl | 2,5-Dimethyl-3,6-dichloro-... | " |
| 39 | —NH— | " | " | Methyl | Chlor | 2-Methyl-3,5,6-trichloro-... | " |
| 40 | —NH— | " | $—CH(CH_3)—CH_2—$ | Chlor | Chlor | 2,3,5,6-Tetrachloro-... | " |
| 41 | —NH— | " | " | Brom | Brom | 2,3,5,6-Tetrabromo-... | " |
| 42 | —NH— | " | 1,4-Butylen | Chlor | Chlor | 2,3,5,6-Tetrachloro-... | " |
| 43 | —NH— | " | 1,6-Hexylen | Chlor | Chlor | " | " |
| 44 | —NH— | " | 2-Sulfato-1,3-propylen | Chlor | Chlor | " | " |

| Bsp. | Verbindung entsprechend Formel (1A) | | | | | Verbindung (5) = . . . 1,4-benzo-chinon | Farbton |
|---|---|---|---|---|---|---|---|
| | Rest B | Rest T | Rest W* | $X^1$ ist . . . | $X^2$ ist . . . | | |
| 45 | —NH— | β-Sulfoethyl | 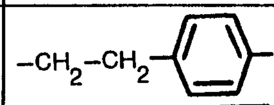 | Chlor | Chlor | 2,3,5,6-Tetrachloro-. . . | rotstichig blau (13) |
| 46 | —NH— | ″ | 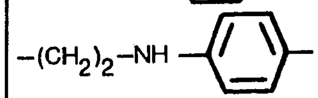 | Chlor | Chlor | ″ | ″ |
| 47 | —NH— | ″ | 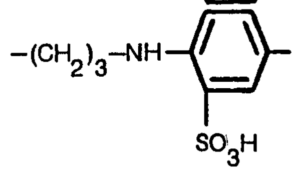 | Chlor | Chlor | ″ | ″ |
| 48 | —NH— | ″ | Sulfophen-1,4-ylen | Chlor | Chlor | ″ | grünstichig blau (15) |
| 49 | —NH— | ″ | 1,5-Pentylen | Chlor | Chlor | ″ | rotstichig blau (13) |
| 50 | —NH— | ″ | $—CH(C_2H_5)—CH_2—$ | Chlor | Chlor | ″ | |
| 51 | —NH— | ″ | $—CH(CH_3)—CH_2—CH_2—$ | Chlor | Chlor | ″ | ″ |
| 52 | —NH— | ″ | 2-Methyl-1,3- propylen | Chlor | Chlor | ″ | ″ |
| 53 | —NH— | ″ | 2-Methoxy-1,3- propylen | Chlor | Chlor | ″ | ″ |
| 54 | —NH— | ″ | $—(CH_2)_3—O—(CH_2)_2—O—(CH_2)_3—$ | Chlor | Chlor | ″ | ″ |
| 55 | —NH— | ″ | $—(CH_2)_2—SO_2—(CH_2)_2—$ | Chlor | Chlor | ″ | ″ |

| Bsp. | Verbindung entsprechend Formel (1A) Rest B | Rest T | Rest W* | X[1] ist . . . | X[2] ist . . . | Verbindung (5) = . . . . 1,4-benzo-chinon | Farbton |
|---|---|---|---|---|---|---|---|
| 56 | —NH— | β-Sulfoethyl | 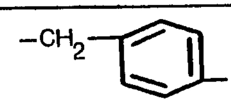 | Chlor | Chlor | 2,3,5,6-Tetrachloro-. . . | rotstichig blau (13) |
| 57 | —NH— | " | 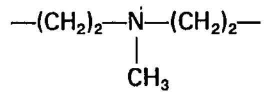 | Chlor | Chlor | " | " |
| 58 | —NH— | " | 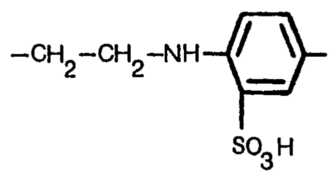 | Chlor | Chlor | " | " |
| 59 | —NH— | " | 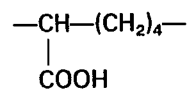 | Chlor | Chlor | " | " |
| 60 | —NH— | " | $—(CH_2)_3—NH—(CH_2)_3—$ | Chlor | Chlor | " | " |
| 61 | —NH— | " | 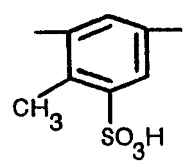 | Chlor | Chlor | " | grünstichig blau (15) |
| 62 | —NH— | " | 1,3-Phenylen | Chlor | Chlor | " | " |
| 63 | —O— | " | 1,3-Propylen | Brom | Brom | 2,3,5,6-Tetrabromo-. . . | blaustichig rot (9) |
| 64 | —O— | " | " | Chlor | Chlor | 2,3,5,6-Tetra-chloro-. . . | " |
| 65 | —O— | " | 1,2-Ethylen | Chlor | Chlor | " | " |

| Bsp. | Verbindung entsprechend Formel (1A) Rest B | Rest T | Rest W* | X¹ ist . . . | X² ist . . . | Verbindung (5) = . . . 1,4-benzo-chinon | Farbton |
|---|---|---|---|---|---|---|---|
| 66 | —NH— | Methyl | 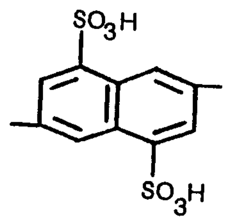 | Chlor | Chlor | 2,3,5,6-Tetrachloro-. . . | grünstichig blau (15) |
| 67 | —NH— | Methyl | 2-Sulfato-1,3-propylen | Chlor | Chlor | " | rotstichig blau (13) |
| 68 | —NH— | Methyl | $-(CH_2)_2-NH-$ 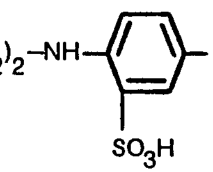 | Chlor | Chlor | " | " |
| 69 | —NH— | β-Carboxyethyl | " | Chlor | Chlor | " | " |
| 70 | —NH— | 4-Sulfobenzyl | 1,3-Propylen | Chlor | Chlor | " | " |

**Patentansprüche**

1. Wasserlösliche Triphendioxazin-Verbindung entsprechend der allgemeinen Formel (I)

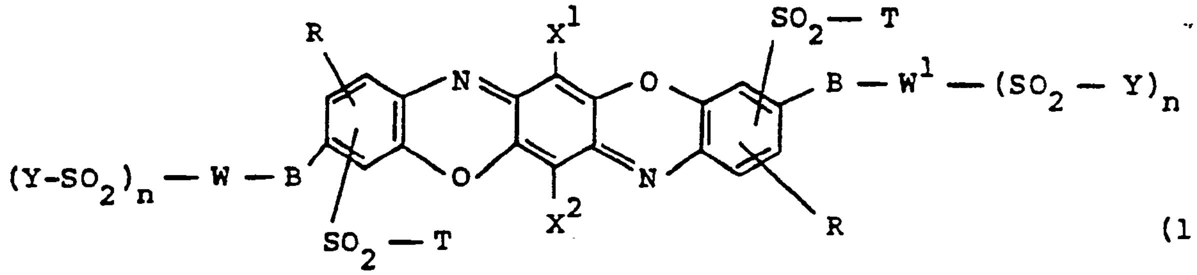

(1)

in welcher bedeuten:

T ist eine gegebenenfalls substituierte Alkylgruppe von 1 bis 6 C-Atomen, ausgenommen ein Rest der nachstehend definierten Gruppe Y, wobei die Alkylgruppe noch durch Heterogruppen, die aus Gruppen der Formeln —O—, —S—, —NH— und —N(R')— mit R' der nachstehend angegebenen Bedeutung ausgewählt sind, unterbrochen sein kann, oder ist eine gegebenenfalls substituierte Arylgruppe;

B ist ein Sauerstoff-oder Schwefelatom oder eine Aminogruppe der Formel —NH— oder —N(R')—, in welcher R' eine Alkylgruppe von 1 bis 6 C-Atomen, ist die substituiert sein kann;

W ist ein bivalenter, gegebenenfalls substituierter aliphatischer, gegebenenfalls durch Alkyl substituierter ($C_5$—$C_{10}$)-cycloaliphatischer, gegebenenfalls durch Alkyl substituierter aliphatisch-($C_5$—$C_8$)-cycloaliphatischer, gegebenenfalls substituierter araliphatischer oder gegebenenfalls substituierter aromatisch-carbocyclischer Rest oder ein mit einem dieser Reste kombinierter Benzotriazolrest, wobei die aliphatischen Reste in W durch Heterogruppen, unterbrochen sein können, die aus den Gruppen —O—, —S—, —$SO_2$—, —CO—, 1.4-Piperidino, —NH— und —N($R^o$)— worin $R^o$ eind der Bedeutungen von R' hat oder eine Alkanoylgruppe von 2 bis 5 C-Atomen, ist, ausgewählt sind, und/oder aliphatische und Arylreste durch eine solche Heterogruppe miteinander verbunden sein können;

$W^1$ hat eine für W angegebene Bedeutung und ist mit W gleich oder von W verschieden;

R ist ein Wasserstoffatom oder ein Alkyl von 1 bis 6 C-Atomen ein Alkoxy von bis 5 C-Atomen, ein Halogen, Carboxy oder Sulfo;

$X^1$ ist ein Wasserstoffatom oder ein Halogenatom, eine Cycloalkylgruppe von 5 bis 8 C-Atomen, eine Aralkyloxygruppe, eine Alkoxygruppe von 1 bis 4 C-Atomen, eine Aryloxygruppe, eine Alkylgruppe von 1 bis 4 C-Atomen, eine Arylgruppe, eine Aralkylgruppe, eine Cyangruppe, eine Carboxygruppe, eine Carbalkoxygruppe von 2 bis 5 C-Atomen, eine Arylaminogruppe, eine Carbamoylgruppe, eine N-Alkyl-carbamoyl-Gruppe oder N,N -Dialkyl-carbamoyl-Gruppe mit Alkylresten von jeweils 1 bis 4 C-Atomen, eine N-Aryl-carbamoyl-Gruppe, eine Alkanoylaminogruppe von 2 bis 5 C-Atomen, oder eine Aroylaminogruppe, wobei die Arylreste in diesen genannten Substituenten bevorzugt Phenylreste sind, die noch durch 1 oder 2 Substituenten aus der Gruppe Halogen, Nitro, Alkyl von 1 bis 4 C-Atomen, Alkoxy von 1 bis 4 C-Atomen, Carboxy und Sulfo substituiert sein können;

$X^2$ is mit $X^1$ gleich oder von $X^1$ verschieden und hat eine der für $X^1$ angegebenen Bedeutungen;

die Gruppe —$SO_2$—T steht bevorzugt in ortho-Stellung zur Gruppe —B—W—($SO_2$—Y)$_n$ bzw. —B—$W^1$—($SO_2$—y)$_n$ gebunden;

n ist die Zahl 1 oder 2;

Y ist die Vinylgruppe oder eine Ethylgruppe, die in ß-Stellung einen durch ein Alkali eliminierbaren Substituenten enthält;

von den Sulfo- und Sulfatogruppen, die im Molekül (1) enthalten sein können, enthält das Molekül (1) zwingend mindestens eine, bevorzugt mindestens zwei davon.

2. Verbindung nach Anspruch 1, dadurch gekennzeichnet, daß R ein Wasserstoffatom ist.

3. Verbindung nach Anspruch 1 oder 2, dadurch gekennzeichnet, daß B eine Aminogruppe —NH— ist.

4. Verbindung nach Anspruch 1, 2 oder 3, dadurch gekennzeichnet, daß W bzw. $W^1$ eine β-(p-Phenylen)-ethylen-Gruppe oder eine 4-Methoxy-1,3-phenylen- oder eine 4-(β-Sulfatoethylamino)-1,3-phenylen- oder eine 4-(β-Sulfoethylamino)-1,3-phenylen-Gruppe ist.

5. Verbindung nach Anspruch 1, 2 oder 3, dadurch gekennzeichnet, daß W ein bivalenter Rest der allgemeinen Formel (2B)

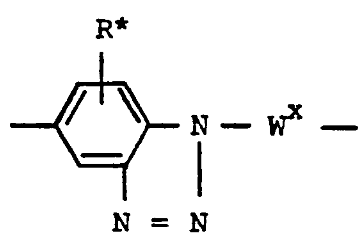

(2B)

und $W^1$ ein bivalenter Rest der allgemeinen Formel (2A)

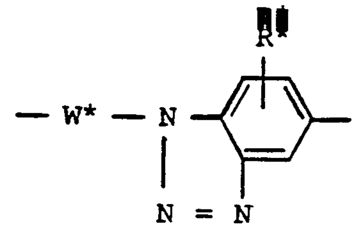

(2A),

ist, in welchen die beiden R*, zueinander gleich oder voneinander verschieden, jedes ein Wasserstoffatom, eine Alkylgruppe von 1 bis 4 C-Atomen, eine Alkoxygruppe von 1 bis 4 C-Atomen, ein Halogenatom, eine Carboxygruppe oder eine Sulfogruppe bedeutet und $W^x$ und W*, zueinander gleich oder voneinander verschieden, jedes einen bivalenten, gegebenenfalls substituierten aliphatischen, gegebenenfalls durch Alkyl substituierten ($C_5$—$C_{10}$)-cycloaliphatischen, gegebenenfalls durch Alkyl substituierten aliphatisch-($C_5$—$C_8$)-cycloaliphatischen, gegebenenfalls substituierten araliphatischen oder gegebenenfalls substituierten aromatisch-carbocyclischen Rest bedeuten, wobei die aliphatischen Reste in W* bzw. $W^x$ durch Heterogruppen unterbrochen sein können, die aus den Gruppen der Formeln —O—, —S—, —$SO_2$—, —CO—, 1,4-Piperidino, —NH— und —N($R^o$)—, in welcher $R^o$ eine Alkylgruppe von 1 bis 6 C-Atomen ist, die substituiert sein kann, oder eine Alkanoylgruppe von 2 bis 5 C-Atomen ist, ausgewählt sind, und/oder wobei aliphatische und Arylreste durch eine solche Heterogruppe miteinander verbunden sein können.

6. Verbindung nach Anspruch 5, dadurch gekennzeichnet, daß W* und $W^x$ beide den 1,2-Ethylen- oder 1,3-Propylenrest bedeuten.

7. Verbindung nach einem der Ansprüche 1 bis 6, dadurch gekennzeichnet, daß T eine β-Sulfoethyl-Gruppe ist.

8. Verbindung nach einem der Ansprüche 1 bis 6, dadurch gekennzeichnet, daß T eine Alkylgruppe von 2 oder 3 C-Atomen ist, die durch eine Sulfo- oder Carboxygruppe substituiert ist.

9. Verbindung nach einem der Ansprüche 1 bis 8, dadurch gekennzeichnet, daß $X^1$ und $X^2$ beide für ein Chlor- oder ein Bromatom stehen

10. Verbindung nach einem der Ansprüche 1 bis 8, dadurch gekennzeichnet, daß $X^1$ und $X^2$ beide für ein Chloratom stehen.

11. Verbindung nach einem der Ansprüche 1 bis 10, dadurch gekennzeichnet, daß Y die β-Sulfatoethyl-Gruppe bedeutet.

12. Verbindung nach Anspruch 1 entsprechend der allgemeinen Formel (1a)

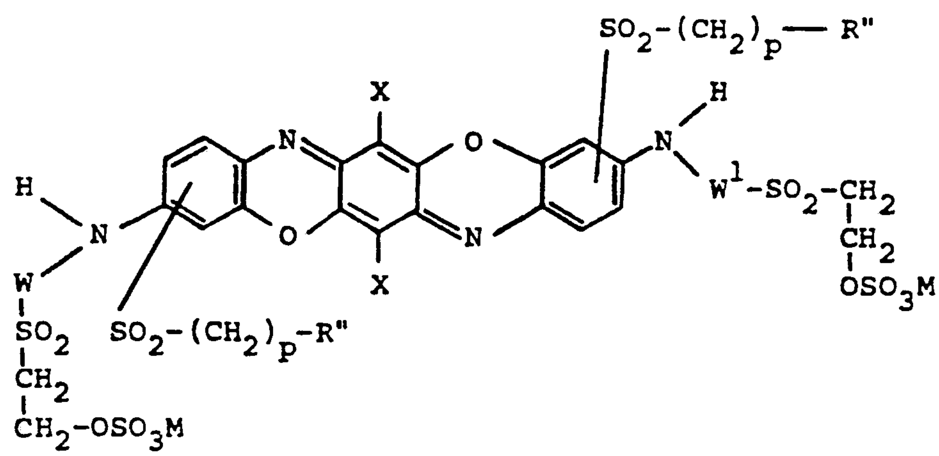

(1a)

in welcher

X für ein Bromatom oder bevorzugt ein Chloratom steht,

M ein Wasserstoffatom oder bevorzugt ein Alkalimetallatom, wie insbesondere Natrium, ist,

p für die Zahl 2 oder 3, bevorzugt 2, steht,

R'' eine Sulfo-, Sulfato- oder Carboxy, bevorzugt Sulfogruppe bedeutet, im Falle von p = 2 jedoch keine Sulfatogruppe bedeutet, und

$W^1$ einen Phenylenrest, bevorzugt meta-Phenylenrest, darstellt, der durch Methyl, Ethyl, Chlor,

Methoxy oder Ethoxy substituiert sein kann, oder eine Gruppe der Formel (2a), (2b) oder (2c)

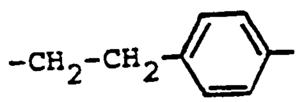
(2a)

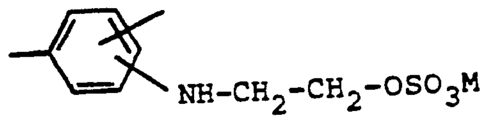
(2b)

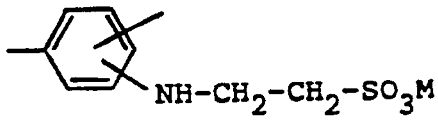
(2c)

mit M der obengenannten Bedeutung ist, wobei in den Formeln (2b) und (2c) der Phenylenrest bevorzugt ein meta-Phenylenrest ist und die Aminogruppe bevorzugt in para-Stellung zur anderen, dem Formelrest B entsprechenden Aminogruppe gebunden ist, und

W einen solchen für $W^1$ angegebenen, jedoch "spiegelbildlich" angeordneten Rest bedeutet.

13. Verfahren zur Herstellung der in Anspruch 1 genannten und definierten Verbindungen, dadurch gekennzeichnet, daß man eine Verbindung der allgemeinen Formel (3)

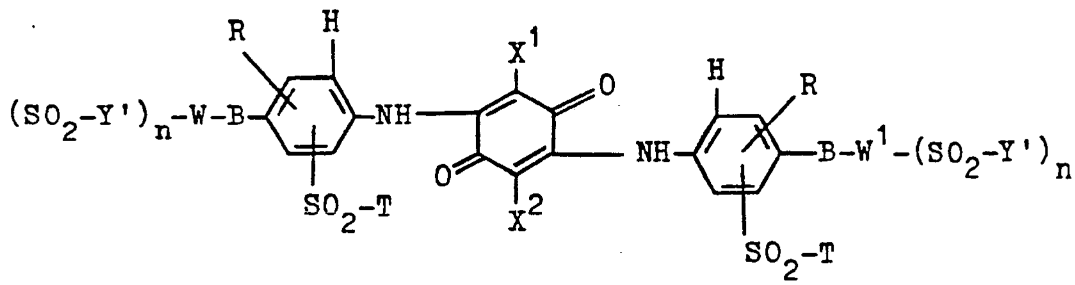
(3)

(in welcher Y' die Vinylgruppe, die β-Hydroxyethyl-Gruppe oder eine Ethylgruppe, die in β-Stellung einen durch ein Alkali eliminierbaren Substituenten enthält, und n, R, B, W, $W^1$, T, $X^1$ und $X^2$ die in Anspruch 1 genannten Bedeutungen haben, wobei substituierte Alkylgruppen in diesen Resten auch hydroxysubstituierte Alkylgruppen sein können und die Gruppen $—SO_2—T$ bevorzugt in ortho-Stellung zur Gruppe $—B—W—(SO_2—Y')_n$ bzw. $—B—W^1—(SO_2—Y')_n$ gebunden sind) in saurem Medium und vorzugsweise in Gegenwart eines Oxidationsmittels zum Triphendioxazin cyclisiert, wobei man gegebenenfalls vor oder gleichzeitig mit der Cyclisierung oder erst nach der Cyclisierungsreaktion gegebenenfalls vorhandene Hydroxyalkylgruppen mittels eines Sulfatisierungs- oder Phosphatierungsmittels zur entsprechenden β-Sulfato-alkyl- bzw. β-Phosphatoalkyl-Gruppen verestert und gegebenenfalls gleichzeitig Sulfogruppen in Arylreste einführt.

14. Verfahren nach Anspruch 13, dadurch gekennzeichnet, daß man den Ringschluß in Gegenwart von Peroxodisulfat durchführt.

15. Verwendung der in Anspruch 1 genannten und definierten Verbindungen der allgemeinen Formel (1) oder der nach Anspruch 13 hergestellten Verbindungen der allgemeinen Formel (1) zum Färben (einschließlich Bedrucken) von hydroxy- und/oder carbonamidgruppenhaltigem Material, insbesondere Fasermaterial.

16. Verfahren zum Färben (einschließlich Bedrucken) von hydroxy- und/oder carbonamidgruppenhaltigem Material, insbesondere Fasermaterial, bei welchem man einen Farbstoff auf das Material aufbringt oder in das Material einbringt und ihn mittels Wärme und/oder mittels eines säurebindenden Mittels fixiert, dadurch gekennzeichnet, daß man als Farbstoff eine Verbindung der in Anspruch 1 genannten und definierten allgemeinen Formel (1) einsetzt.

17. Verbindung der allgemeinen Formel (4)

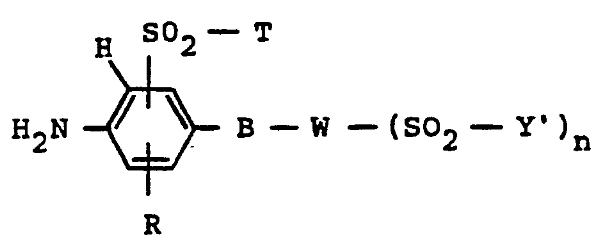
(4)

in welcher Y' die Vinylgruppe, die β-Hydroxyethyl-Gruppe oder eine Ethylgruppe, die in β-Stellung einen durch ein Alkali eliminierbaren Substituenten enthält, sowie R, T, B, W und n die in Anspruch 1 genannten Bedeutungen haben und die Gruppe $-SO_2-T$ bevorzugt in ortho-Stellung zur Gruppe $-B-W-(SO_2-Y')_n$ gebunden steht.

18. Verbindung der allgemeinen Formel (8)

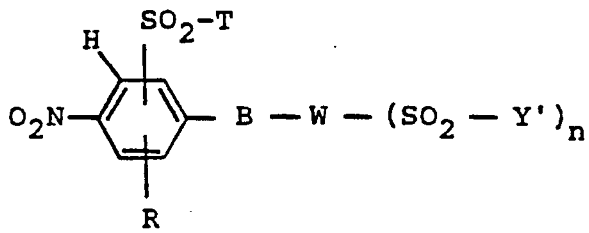

(8)

in welcher Y' die Vinylgruppe, die β-Hydroxyethyl-Gruppe oder eine Ethylgruppe, die in β-Stellung einen durch ein Alkali eliminierbaren Substituenten enthält, sowie R, T, B, W und n die in Anspruch 1 genannten Bedeutungen haben und die Gruppe $-SO_2-T$ bevorzugt in ortho-Stellung zur Gruppe $-B-W-(SO_2-Y')_n$ gebunden steht.

19. Verbindung nach Anspruch 17 oder 18, dadurch gekennzeichnet, daß W ein bivalenter Rest der allgemeinen Formel (2B)

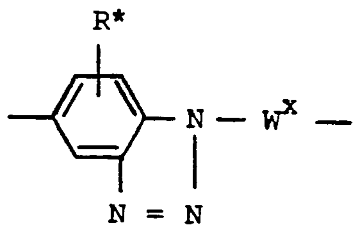

(2B)

und $W^1$ ein bivalenter Rest der allgemeinen Formel (2A)

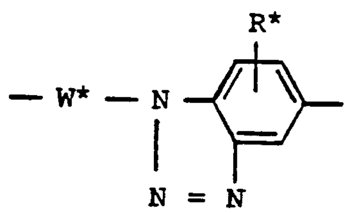

(2A),

ist, in welchen die beiden R*, zueinander gleich oder voneinander verschieden, jedes ein Wasserstoffatom, eine Akylgruppe von 1 bis 4 C-Atomen, eine Alkoxygruppe von 1 bis 4 C-Atomen, ein Halogenatom, eine Carboxygruppe oder eine Sulfogruppe bedeutet und $W^x$ und W*, zueinander gleich oder voneinander verschieden, jedes einen bivalenten, gegebenenfalls substituierten aliphatischen, gegebenenfalls durch Alkyl substituierten ($C_5$—$C_{10}$)-cycloaliphatischen, gegebenenfalls durch Alkyl substituierten aliphatisch-($C_5$—$C_8$)-cycloaliphatischen, gegebenenfalls substituierten araliphatischen oder gegebenenfalls substituierten aromatisch-carbocyclischen Rest bedeuten, wobei die aliphatischen Reste in W* bzw.

$W^x$ durch Heterogruppen unterbrochen sein können, die aus den Gruppen der Formeln —O—, —S—, $-SO_2-$, —CO—, 1,4-Piperidino, —NH— und —N(R°)—, in welcher R° eine Alkylgruppe von 1 bis 6 C-Atomen ist, die substituiert sein kann, oder eine Alkanoylgruppe von 2 bis 5 C-Atomen ist, ausgewählt sind, und/oder wobei aliphatische und Arylreste durch eine solche Heterogruppe miteinander verbunden sein können.

20. Verfahren zur Herstellung einer in Anspruch 17 angegebenen und definierten Verbindung der allgemeinen Formel (4) oder einer in Anspruch 18 angegebenen und definierten Verbindung der allgemeinen Formel (8), dadurch gekennzeichnet, daß man eine Verbindung der allgemeinen Formel (6)

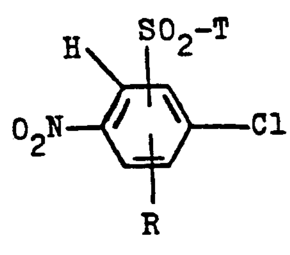

(6)

in welcher R und T die in Anspruch 17 angegebenen Bedeutungen haben, mit einer Aminoverbindung der allgemeinen Formel (7)

$$H—B—W—(SO_2—Y')_n \quad (7)$$

in welcher B, W, Y' und n die in Anspruch 17 genannten Bedeutungen haben, unter Zusatz eines basischen, säurebindenden Mittels bei einer Temperatur zwischen 20 und 120°C umsetzt und gegebenenfalls in der so erhaltenen Nitroverbindung der allgemeinen Formel (8) die Nitrogruppe zur Aminogruppe reduziert.

21. Verwendung der Verbindung der allgemeinen Formel (4) von Anspruch 17 oder der Verbindung der allgemeinen Formel (8) von Anspruch 18 zur Synthese von Farbstoffen, insbesondere von Triphendioxazin-Verbindungen der allgemeinen Formel (1) gemäß Anspruch 1.

**Revendications**

1. Triphénodioxazine hydrosoluble, répondant à la formule générale (1):

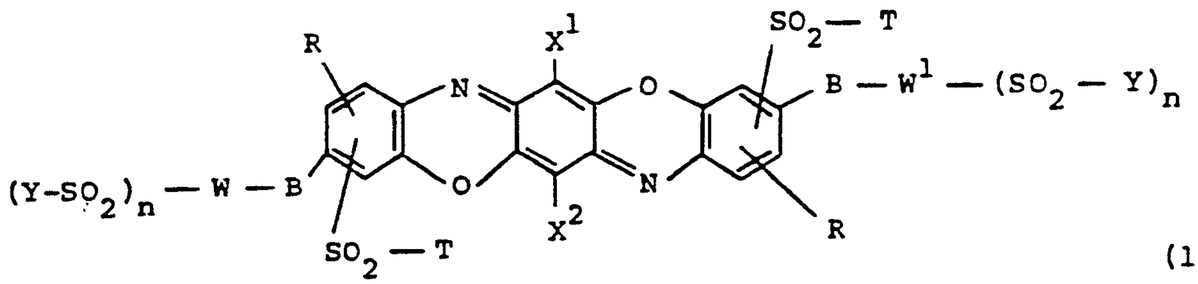

(1)

dans laquelle:

T représente un groupe alkyle ayant 1 à 6 atomes de carbone, éventuellement substitués, à l'exclusion d'un reste Y et répondant à la définition ci-après de ce groupe Y, le groupe alkyle pouvant encore être interrompu par des hétéro-groupes, choisis parmi des groupes répondant aux formules —O—, —S—, —NH— et —N(R')—, où R' a le sens indiqué ci-après, ou bien il représente un groupe aryle éventuellement substitué);

B représente un atome d'oxygène ou de soufre ou un groupe amino de formule —NH— ou —N(R')—, dans laquelle R' est un groupe alkyle ayant 1 à 6 atomes de carbone, qui peut être substitué;

W représente un reste divalent aliphatique éventuellement substitué, cycloaliphatique en $C_5$ à $C_{10}$ (éventuellement substitué par un groupe alkyle), aliphatique-cycloaliphatique (ce dernier en $C_5$ à $C_8$) éventuellement substitué par un groupe alkyle, un reste araliphatique éventuellement substitué ou carbocyclique aromatique éventuellement substitué ou un reste benzotriazole combiné à l'un de ces restes, les restes aliphatiques présents dans W pouvant être interrompus par des hétéro-groupes (choisis parmi les groupes —O—, —S—, —$SO_2$—, —CO—, pipéridino-1,4, —NH— et —N(R°)—, où R° a l'un des sens de R', ou un groupe alcanoyle ayant 2 à 5 atomes de carbone) et/ou des restes aliphatiques et aryles pouvant être reliés l'un à l'autre à l'aide d'un tel hétéro-groupe;

$W^1$ a un sens indiqué pour W et est identique à W ou en diffère;

R représente un atome d'hydrogène ou un groupe alkyle ayant 1 à 6 atomes de carbone, un groupe alcoxy ayant 1 à 5 atomes de carbone, un halogène, un groupe carboxy ou sulfo;

$X^1$ représente un atome d'hydrogène ou un atome d'halogène, un groupe cycloalkyle ayant 5 à 8 atomes de carbone, un aralkyloxy, un groupe alcoxy ayant 1 à 4 atomes de carbone, un groupe aryloxy, un groupe alkyle ayant 1 à 4 atomes de carbone, un groupe aryle, un groupe aralkyle, un groupe cyano, un groupe carboxy, un groupe carbalcoxy ayant 2 à 5 atomes de carbone, un groupe arylamino, un groupe carbamoyle, un groupe N-alkyl-carbamoyle ou un groupe N,N-dialkyl-carbamoyle comportant des restes alkyles ayant chacun 1 à 4 atomes de carbone, un groupe N-aryl-carbamoyle, un groupe alcanoylamino ayant 2 à 5 atomes de carbone ou un groupe aroylamino, les restes aryles présents dans ces substituants cités étant de préférence des restes phényles, pouvant porter encore eux-mêmes un ou deux substituants choisis parmi un atome d'halogène, un groupe nitro, alkyle ayant 1 à 4 atomes de carbone, alcoxy ayant 1 à 4 atomes de carbone, carboxy et sulfo;

$X^2$ est identique à $X^1$ ou en diffère et a l'un des sens indiqués pour $X^1$;

le groupe —$SO_2$—T est de préférence fixé en position ortho par rapport au groupe —B—W—($SO_2$—Y)$_n$ ou —B—$W^1$—($SO_2$—Y)$_n$;

n est un nombre valant 1 ou 2;

Y représente le groupe vinyle ou un groupe éthyle, qui contient en position 2 un substituant pouvant être éliminé à l'aide d'une substance alcaline; parmi les groupes sulfo et sulfato pouvant être contenus dans la molécule (1), cette molécule (1) en contient obligatoirement au moins un, et de préférence au moins deux.

2. Composé selon la revendication 1, caractérisé en ce que R est un atome d'hydrogène.

3. Composé selon la revendication 1 ou 2, caractérisé en ce que B est un groupe amino —NH—.

4. Composé selon la revendication 1, 2 ou 3, caractérisé en ce que W ou $W^1$ représente un groupe (p-phénylène)-2 éthylène ou un groupe méthoxy-4 phénylène-1,3 ou un groupe (sulfato-2 éthylamino)-4 phénylène-1,3 ou un groupe (sulfo-2 éthylamino)-4 phénylène-1,3.

5. Composé selon l'une des revendications 1, 2 ou 3, caractérisé en ce que W est un reste divalent répondant à la formule générale (2B):

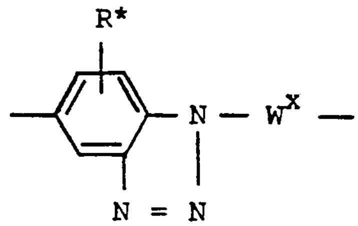

(2B)

et $W^1$ est un reste divalent répondant à la formule générale (2A):

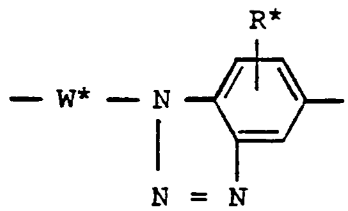

(2A),

dans lesquelles les deux symboles R* peuvent représenter des groupes identiques ou différents, et sont chacun un atome d'hydrogène, un groupe alkyle ayant 1 à 4 atomes de carbone, un groupe alcoxy ayant 1 à 4 atomes de carbone, un atome d'halogène, un groupe carboxy ou un groupe sulfo, et $W^x$ et W*, identiques ou différents, représentent chacun un reste divalent, aliphatique éventuellement substitué, cycloaliphatique (en $C_5$ à $C_{10}$) éventuellement substitué par un groupe alkyle, aliphatique-cycloaliphatique (ce dernier en $C_5$ à $C_8$) éventuellement substitué par un groupe alkyle, araliphatique éventuellement substitué, les restes aliphatiques présents dans W* ou $W^x$ pouvant être interrompus par des hétérogroupes, (choisis parmi des groupes de formule —O—, —S—, —$SO_2$—, —CO—, pipéridino-1,4, —NH—, et —N(R°)—, où R° représente un groupe alkyle ayant 1 à 6 atomes de carbone, pouvant être substitué, ou un groupe alcanoyle ayant 2 à 5 atomes de carbone) et/ou les restes aliphatiques et aryles pouvant être reliés l'un à l'autre à l'aide d'un tel hétérogroupe.

6. Composé selon la revendication 5, caractérisé en ce que W* et $W^x$ représentent tous deux le reste éthylène-1,2 ou propylène-1,3.

7. Composé selon l'une des revendications 1 à 6, caractérisé en ce que T représente un groupe sulfo-2 éthyle.

8. Composé selon l'une des revendications 1 à 6, caractérisé en ce que T représente un groupe alkyle ayant 2 ou 3 atomes de carbone, qui est substitué par un groupe sulfo ou carboxy.

9. Composé selon l'une des revendications 1 à 8, caractérisé en ce que $X^1$ et $X^2$ représentent tous deux chacun un atome de chlore ou un atome de brome.

10. Composé selon l'une des revendications 1 à 8, caractérisé en ce que $X^1$ et $X^2$ représentent tous deux chacun un atome de chlore.

11. Composé selon l'une des revendications 1 à 10, caractérisé en ce que Y représente le groupe sulfato-2 éthyle.

12. Composé selon la revendication 1, répondant à la formule générale (1a):

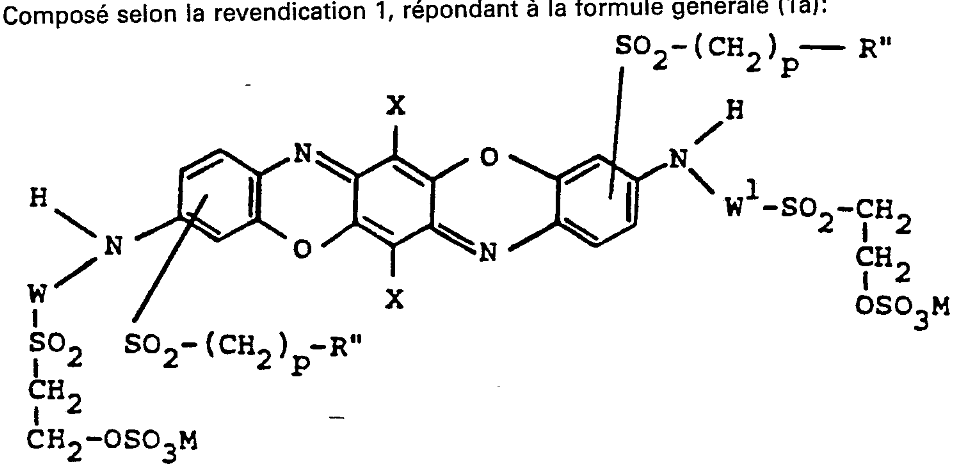

(1a)

dans laquelle:

X représente un atome de brome ou de préférence un atome de chlore,

M représente un atome d'hydrogène ou, de préférence, un atome de métal alcalin comme, notamment, le sodium,

P est un nombre valant 2 ou 3, de préférence 2,

R'' représente un groupe sulfo, sulfato ou carboxy, de préférence un groupe sulfo mais, si p vaut 2, il ne représente pas de groupe sulfato, et

$W^1$ représente un reste phénylène, de préférence un reste méta-phénylène, (pouvant être substitué par un groupe méthyle, éthyle, chloro, méthoxy ou éthoxy), ou un groupe répondant à l'une des formules (2a), (2b) ou (2c):

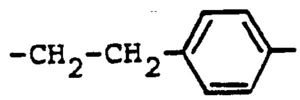 (2a)

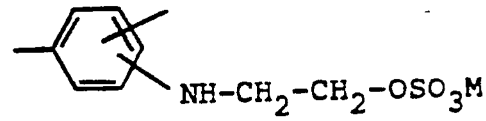 (2b)

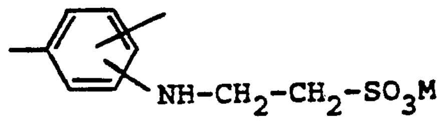 (2c)

dans lesquelles M a le sens précité et, dans les formules (2b) et (2c), le reste phénylène est de préférence un reste méta-phénylène, et le groupe amino est de préférence fixé en para par rapport à d'autres groupes amino présents dans le reste B, et

W est un reste tel que ceux indiqués pour $W^1$, mais en constitue "l'image dans un miroir".

13. Procédé pour la préparation des composés cités et définis à la revendication 1, caractérisé en ce qu'on soumet un composé de formule générale (3):

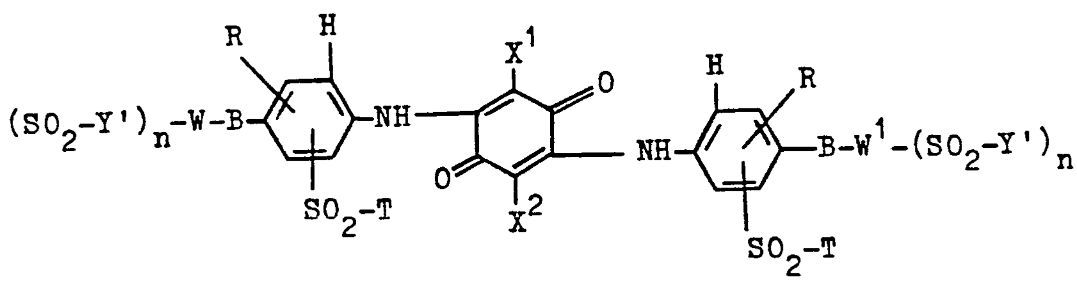 (3)

(dans laquelle Y' représente le groupe vinyle, le groupe hydroxy-2 éthyle ou un groupe éthyle contenant en position 2 un substituant pouvant être éliminé à l'aide d'une substance alcaline, et n, R, B, W, $W^1$, T, $X^1$ et $X^2$ ont les sens indiqués à la revendication 1, les groupes alkyles substitués présents dans ces restes pouvant également être des groupes alkyles à substituant(s) hydroxy, et les groupes $—SO_2—T$ étant de préférence fixés en position ortho par rapport au groupe $—B—W—(SO_2—Y')_n$ ou $—B—W^1—(SO_2—Y')_n$) à une cyclisation effectuée en milieu acide et de préférence en présence d'un oxydant pour obtenir une triphénodioxazine, et l'on estérifie éventuellement, à l'aide d'un agent de sulfatation, ou de phosphatation, avant ou pendant la cyclisation ou seulement après la réaction de cyclisation, des groupes hydroxyles éventuellement présents pour obtenir des groupes sulfato-2 alkyles ou phosphato-2 alkyles correspondants, et l'on introduit éventuellement en même temps des groupes sulfo dans des restes aryles.

14. Procédé selon la revendication 13, caractérisé en ce qu'on conduit la cyclisation en présence d'un peroxodisulfate.

15. Utilisation des composés de formule générale (1), cités et définis à la revendication 1, ou des composés de formule générale (1), produits selon la revendication 13, pour teindre (ce qui comprend l'impression) une matière contenant les groupes hydroxyles et/ou des groupes carboxamides, notamment une matière fibreuse.

16. Procédé pour teindre (ce qui comprend l'impression) d'une matière contenant des groupes hydroxyles et/ou carboxamides, en particulier une matière fibreuse, dans lequel on applique un colorant sur la matière ou l'on fait pénétrer ce colorant dans la matière et on l'y fixe à l'aide de la chaleur et/ou à l'aide d'un agent de fixation des acides, procédé caractérisé en ce qu'on utilise comme colorant un composé de formule générale (1) citée et définie à la revendication 1.

17. Composé de formule générale (4):

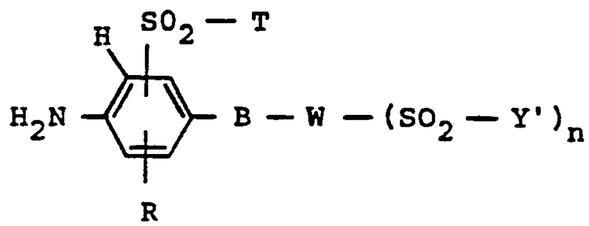

(4)

dans laquelle Y′ représente le groupe vinyle, le groupe hydroxy-2 éthyle ou un groupe éthyle, qui contient en position 2 un substituant éliminable à l'aide d'une substance alcaline, et R, T, B, W et n ont les sens indiqués à la revendication 1, et le groupe $—SO_2—T$ est fixé de préférence en position ortho par rapport au groupe $—B—W—(SO_2—Y')_n$.

18. Composé de formule générale (8):

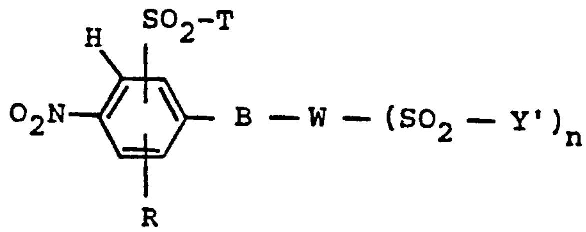

(8)

dans laquelle Y′ représente le groupe vinyle, le groupe hydroxy-2 éthyle ou un groupe éthyle contenant en position 2 un substituant éliminable à l'aide d'une substance alcaline, et R, T, B, W et n ont les sens indiqués à la revendication 1, et le groupe $—SO_2—T$ est fixé de préférence en position ortho par rapport au groupe $—B—W—(SO_2—Y')_n$.

19. Composé selon la revendication 17 ou 18, caractérisé en ce que W représente un reste bivalent répondant à la formule générale (2B):

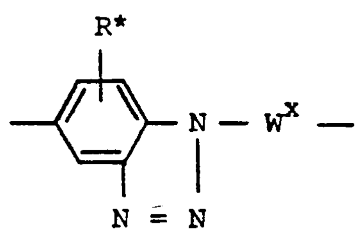

(2B)

et $W^1$ représente un reste bivalent répondant à la formule générale (2A):

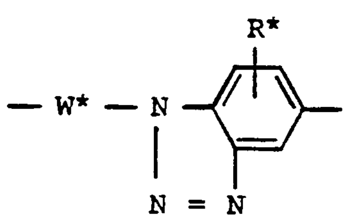

(2A),

dans lesquelles les deux symboles R*, identiques ou différents, représentent chacun un atome d'hydrogène, un groupe alkyle ayant 1 à 4 atomes de carbone, un groupe alcoxy ayant 1 à 4 atomes de carbone, un atome d'halogène, un groupe carboxy ou un groupe sulfo, et $W^x$ et $W^*$, identiques ou différents, représentent chacun un reste divalent, aliphatique éventuellement substitué, cycloaliphatique en $C_5$ à $C_{10}$ éventuellement substitué par un groupe alkyle, aliphatique-cycloaliphatique (ce dernier en $C_5$ à $C_8$) éventuellement substitué par un groupe alkyle, araliphatique éventuellement substitué ou carbocyclique aromatique éventuellement substitué, les restes aliphatiques présents dans $W^*$ ou $W^x$ pouvant être interrompus par des hétéro-groupes (choisis parmi les groupes répondant aux formules —O—, —S—, $—SO_2—$, —CO—, pipéridino-1,4, —NH— et $—N(R^o)—$, où $R^o$ représente un groupe alkyle ayant 1 à 6 atomes de carbone, qui peut être substitué, ou un groupe alcanoyle ayant 2 à 5 atomes de carbone), et/ou dans les restes aliphatiques et aryles pouvant être reliés les uns aux autres à l'aide d'un tel hétéro-groupe.

20. Procédé pour préparer l'un des composés de formule générale (4), cités et définis à la revendication 17, ou un composé de formule générale (8), indiqué et défini à la revendication 18, procédé caractérisé en ce qu'on fait réagir un composé de formule générale (6):

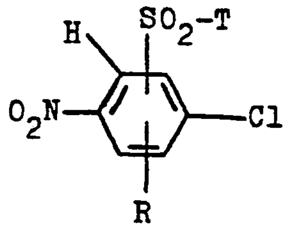

(6)

(dans laquelle R et T ont les sens indiqués à la revendication 17) avec une amine de formule générale (7):

$$H—B—W—(SO_2—Y')_n \quad (7)$$

(dans laquelle B, W, Y' et n ont les sens indiqués à la revendication 17), avec addition d'un agent basique de fixation des acides, en opérant à une température comprise entre 20 et 120°C, et éventuellement, dans le composé nitré de formule générale (8) ainsi obtenu, on réduit le groupe nitro en un groupe amino.

21. Utilisation du composé de formule générale (4) de la revendication 17 ou du composé de formule générale (8) de la revendication 18 pour la synthèse de colorants, en particulier de triphénodioxazines de formule générale (1) selon la revendication 1.

**Claims**

1. A water-soluble triphendioxazine compound corresponding to the formula (1)

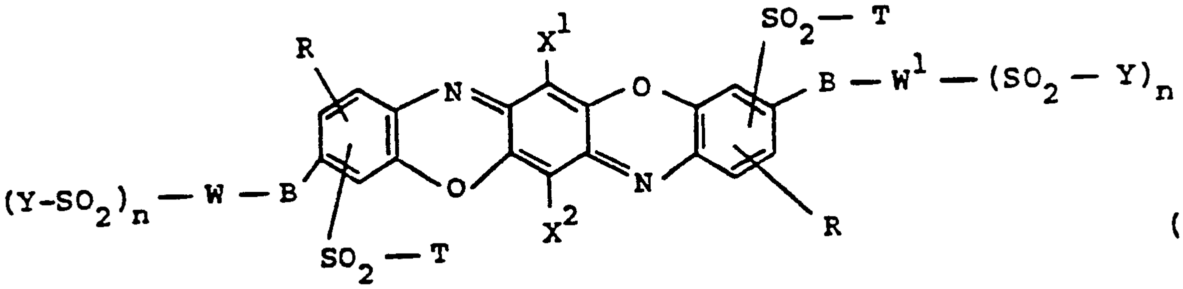

(1)

in which:

T is an optionally substituted alkyl group with 1 to 6 carbon atoms, excluding a radical of the group Y defined below, it also being possible for the alkyl group to be interrupted by hetero groups selected from groups of the formulae —O—, —S—, —NH— and —N(R')—, where R' has the meaning given below, or T is an optionally substituted aryl group;

B is an oxygen or sulfur atom or an amino group of the formula —NH— or —N(R')—, in which R' is an alkyl group with 1 to 6 carbon atoms which can be substituted;

W is a divalent, optionally substituted aliphatic, optionally alkyl-substituted ($C_5$—$C_{10}$)-cycloaliphatic, optionally alkyl-substituted aliphatic-($C_5$—$C_8$)-cycloaliphatic, optionally substituted araliphatic or optionally substituted aromatic-carbocyclic radical or a benzotriazole radical combined with one of these radicals, it being possible for the aliphatic radicals in W to be interrupted by hetero groups selected from the groups —O—, —S—, —$SO_2$—, —CO—, 1,4-piperidino-, —NH— and —N($R^o$), in which $R^o$ has one of the meanings of R' or is an alkanoyl group with 2 to 5 carbon atoms and/or it being possible for aliphatic and aryl radicals to be linked to one another by such a hetero group;

$W^1$ has one of the meanings given for W and is identical to W or different from W;

R is a hydrogen atom or alkyl with 1 to 6 carbon atoms, alkoxy with 1 to 5 carbon atoms, halogen, carboxyl or sulfo;

$X^1$ is a hydrogen atom or a halogen atom, a cycloalkyl group with 5 to 8 carbon atoms, an aralkyloxy group, an alkoxy group with 1 to 4 carbon atoms, an aryloxy group, an alkyl group with 1 to 4 carbon atoms, an aryl group, an aralkyl group, a cyano group, a carboxyl group, a carbalkoxy group with 2 to 5 carbon atoms, an arylamino group, a carbamoyl group, an N-alkyl-carbamoyl group or N,N-dialkyl-carbamoyl group with alkyl radicals with in each case 1 to 4 carbon atoms, an N-aryl-carbamoyl group, an alkanoylamino group with 2 to 5 carbon atoms or an aroylamino group, the aryl radicals in these substituents mentioned preferably being phenyl radicals which can also be substituted by 1 or 2 substituents from the group comprising halogen, nitro, alkyl with 1 to 4 carbon atoms, alkoxy with 1 to 4 carbon atoms, carboxyl and sulfo;

$X^2$ is identical to $X^1$ or different from $X^1$ and has one of the meanings given for $X^1$;

the group $—SO_2—T$ is preferably bonded in the ortho-position relative to the group $—B—W—(SO_2—Y)_n$ or $—B—W^1—(SO_2—Y)_n$;

n is the number 1 or 2;

Y is the vinyl group or an ethyl group which contains, in the β-position, a substituent which can be eliminated by an alkali; of the sulfo and sulfato groups which the molecule (1) can contain, the molecule (1) necessarily contains at least one, and preferably at least two.

2. A compound as claimed in claim 1, in which R is a hydrogen atom.

3. A compound as claimed in either of claims 1 and 2, in which B is an amino group —NH—.

4. A compound as claimed in any one of claims 1, 2 and 3, in which W or $W^1$ is a β-(p-phenylene)-ethylene group or a 4-methoxy-1,3-phenylene or a 4-(β-sulfatoethylamino)-1,3-phenylene or a 4-(β-sulfoethylamino)-1,3-phenylene group.

5. A compound as claimed in any one of claims 1, 2 and 3, in which W is a divalent radical of the formula (2B)

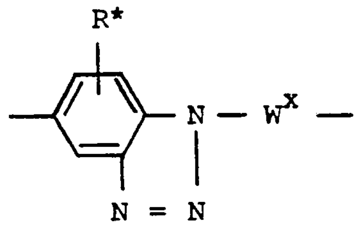

(2B)

and $W^1$ is a divalent radical of the formula (2A)

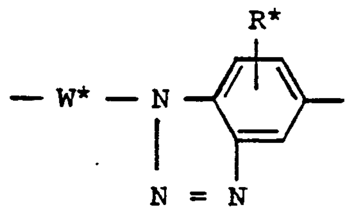

(2A),

in which the two radicals R* are identical to one another or different from one another and each denotes a hydrogen atom, an alkyl group with 1 to 4 carbon atoms, an alkoxy group with 1 to 4 carbon atoms, a halogen atom, a carboxyl group or a sulfo group and $W^x$ and W* are identical to one another or different from one another and each denotes a divalent, optionally substituted aliphatic, optionally alkyl-substituted $(C_5—C_{10})$-cycloaliphatic, optionally alkyl-substituted aliphatic $(C_5—C_8)$-cycloaliphatic, optionally substituted araliphatic or optionally substituted aromatic-carbocyclic radical, it being possible for the aliphatic radicals in W* and $W^x$ to be interrupted by hetero groups selected from the groups of the formulae —O—, —S—, $—SO_2—$, —CO—, 1,4-piperidino, —NH— and $—N(R^o)—$, where $R^o$ is an alkyl group with 1 to 6 carbon atoms which can be substituted, or an alkanoyl group with 2 to 5 carbon atoms, and/or it being possible for aliphatic and aryl radicals to be bonded to one another by such a hetero group.

6. A compound as claimed in claim 5, in which W* and $W^x$ both denote the 1,2-ethylene or 1,3-propylene radical.

7. A compound as claimed in any one of claims 1 to 6, in which T is a β-sulfoethyl group.

8. A compound as claimed in any one of claims 1 to 6, in which T is an alkyl group with 2 or 3 carbon atoms which is substituted by a sulfo or carboxyl group.

9. A compound as claimed in any one of claims 1 to 8, in which $X^1$ and $X^2$ both represent a chlorine or a bromine atom.

10. A compound as claimed in any one of claims 1 to 8, in which $X^1$ and $X^2$ both represent a chlorine atom.

11. A compound as claimed in any one of claims 1 to 10, in which Y denotes the β-sulfatoethyl group.

12. A compound as claimed in claim 1, corresponding to the formula (1a)

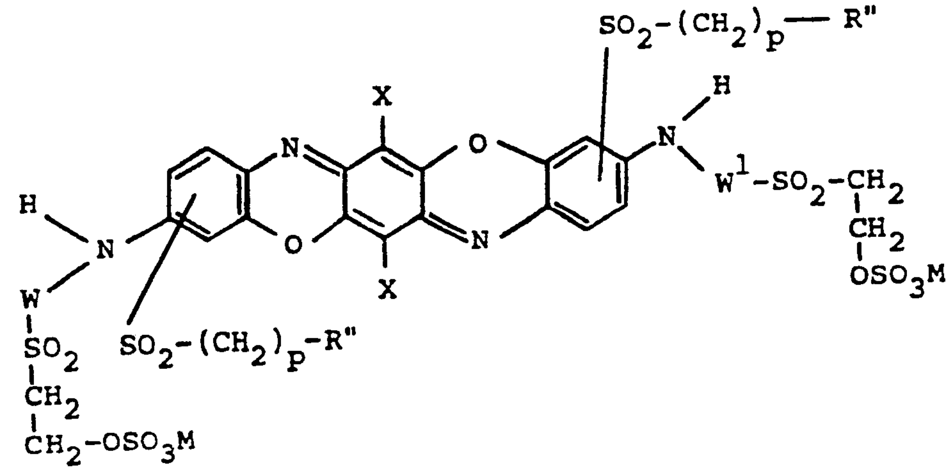

(1a)

in which

X represents a bromine atom or, preferably, a chlorine atom,

M is a hydrogen atom or, preferably, an alkali metal atom, such as, in particular, sodium,

p represents the number 2 or 3, preferably 2,

R″ denotes a sulfo, sulfato or carboxyl group, preferably a sulfo group, but in the case of p = 2 does not denote a sulfato group, and

$W^1$ represents a phenylene radical, preferably a metaphenylene radical, which can be substituted by methyl, ethyl, chlorine, methoxy or ethoxy, or is a group of the formula (2a), (2b) or (2c)

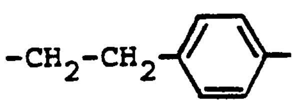

(2a)

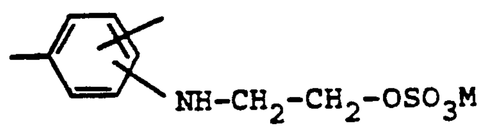

(2b)

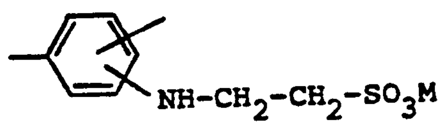

(2c)

where M has the abovementioned meaning, the phenylene radical in the formulae (2b) and (2c) preferably being a meta-phenylene radical and the amino group preferably being bonded in the para-position to the other amino group corresponding to the formula radical B, and

W represents a radical of the type indicated for $W^1$, but arranged in "mirror-image".

13. A process for the preparation of a compound mentioned and defined in claim 1, which comprises cyclizing a compound of the formula (3)

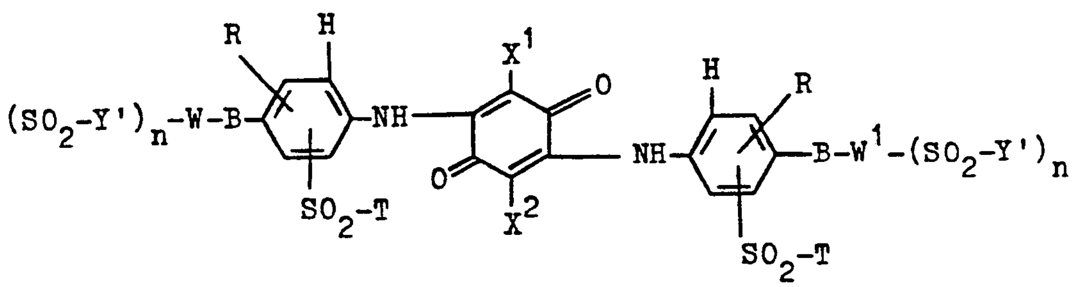

(3)

(in which Y′ is the vinyl group, the β-hydroxyethyl group or an ethyl group which contains, in the β-position, a substituent which can be eliminated by an alkali, and n, R, B, W, $W^1$, T, $X^1$ and $X^2$ have the meanings given in claim 1, it being possible for substituted alkyl groups in these radicals also to be hydroxy-substituted alkyl groups, the groups —$SO_2$—T preferably being bonded in the ortho-position relative to the group —B—W—($SO_2$—Y′)$_n$ and —B—$W^1$—($SO_2$—Y′)$_n$) in an acid medium and preferably in

the presence of an oxidizing agent to give the triphendioxazine, if appropriate, any hydroxyalkyl groups present being esterified into the corresponding β-sulfato-alkyl or β-phosphato-alkyl groups by means of a sulfating or phosphating agent before or at the same time as the cyclization or after the cyclization reaction, and if appropriate sulfo groups simultaneously being introduced in aryl radicals.

14. The process as claimed in claim 13, wherein the cyclization is carried out in the presence of peroxodisulfate.

15. The use of a compound of the formula (1) mentioned and defined in claim 1 or of a compound of the formula (1) prepared as claimed in claim 13 for dyeing (including printing) material containing hydroxyl and/or carboxamide groups, in particular fiber material.

16. A process for dyeing (including printing) material containing hydroxyl and/or carboxamide groups, in particular fiber material, in which a dyestuff is applied to the material or incorporated into the material and is fixed by means of heat and/or by means of an acid-binding agent, which comprises using a compound of the formula (1) mentioned and defined in claim 1 as the dyestuff.

17. A compound of the formula (4)

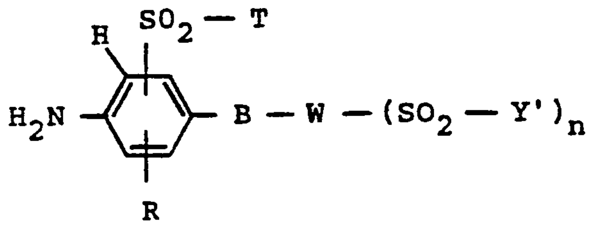 (4)

in which Y' is the vinyl group, the β-hydroxyethyl group or an ethyl group which contains, in the β-position, a substituent which can be eliminated by an alkali, R, T, B, W and n have the meanings given in claim 1 and the group $—SO_2—T$ is preferably bonded in the ortho-position relative to the group $—B—W—(SO_2—Y')_n$.

18. A compound of the formula (8)

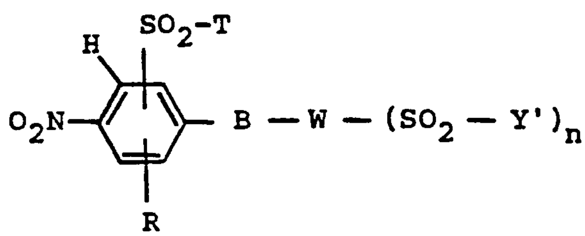 (8)

in which Y' is the vinyl group, the β-hydroxyethyl group or an ethyl group which contains, in the β-position, a substituent which can be eliminated by an alkali, R, T, B, W and n have the meanings given in claim 1 and the group $—SO_2—T$ is preferably bonded in the ortho-position relative to the group $—B—W—(SO_2—Y')_n$.

19. A compound as claimed in either of claims 17 and 18, in which W is a divalent radical of the formula (2B)

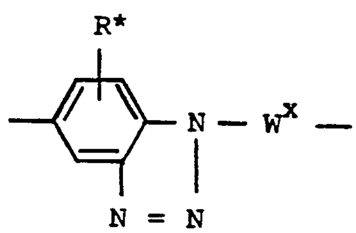 (2B)

and $W^1$ is a divalent radical of the formula (2A)

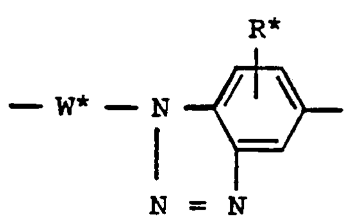 (2A),

in which the two radicals R* are identical to one another or different from one another and each denotes a hydrogen atom, an alkyl group with 1 to 4 carbon atoms, an alkoxy group with 1 to 4 carbon atoms, a halogen atom, a carboxyl group or a sulfo group and $W^x$ and W* are identical to one another or different

from one another and each denotes a divalent, optionally substituted aliphatic, optionally alkyl-substituted ($C_5$—$C_{10}$)-cycloaliphatic, optionally alkyl-substituted aliphatic ($C_5$—$C_8$)-cycloaliphatic, optionally substituted araliphatic or optionally substituted aromatic-carbocyclic radical, it being possible for the aliphatic radicals in W* and $W^x$ to be interrupted by hetero groups selected from groups of the formulae —O—, —S—, —$SO_2$—, —CO—, 1,4-piperidino, —NH— and —N($R^o$)—, where $R^o$ is an alkyl group with 1 to 6 carbon atoms which may be substituted, or an alkanoyl group with 2 to 5 carbon atoms, and/or it being possible for aliphatic and aryl radicals to be bonded to one another by such a hetero group.

20. A process for the preparation of a compound of the formula (4) mentioned and defined in claim 17 or a compound of the formula (8) mentioned and defined in claim 18, which comprises reacting a compound of the formula (6)

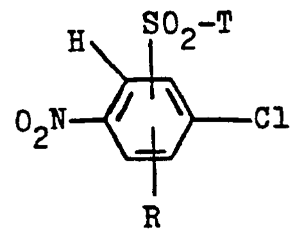

(6)

in which R and T have the meanings given in claim 17, with an amino compound of the formula (7)

$$H—B—W—(SO_2—Y')_n \qquad (7)$$

in which B, W, Y' and n have the meanings given in claim 17, with the addition of a basic acid-binding agent at a temperature between 20 and 120°C and, if desired, reducing the nitro group to the amino group in the nitro compound of the formula (8) thus obtained.

21. The use of a compound of the formula (4) of claim 17 or of a compound of the formula (8) of claim 18 for the synthesis of a dyestuff, in particular a triphendioxazine compound of the formula (1) as claimed in claim 1.